# EUROPÄISCHE PATENTANMELDUNG

(11) **EP 1 780 279 A1**
(43) Veröffentlichungstag der Anmeldung: **02.05.2007**
(21) Anmeldenummer: 06118519.5
(22) Anmeldetag: 13.07.2000
(51) Int. Cl.: C12N 15/12, C12N 15/10, C07K 14/47, C12N 5/10, C12P 21/02, G01N 33/68, A61K 38/17

(54) **Design von Beta-Faltblatt-Proteinen mit spezifischen Bindungseigenschaften**

(30) Priorität: 13.07.1999 DE 19932688
(62) Teilanmeldung aus: 00944034.8
(71) Anmelder: Scil proteins GmbH, 06120 Halle/Saale (DE)
(72) Erfinder: Fiedler, Ulrike, 06114 Halle (DE); Rudolph, Rainer, 06120 Halle (DE)
(74) Vertreter: Behnisch, Werner

(57) **Zusammenfassung**

Die vorliegende Erfindung beschreibt neuartige Beta-Faltblatt-Proteine mit spezifischen Bindungseigenschaften und katalytischen Eigenschaften sowie Verfahren zur Herstellung dieser Proteine.

SEQUENZPROTOKOLL

<110> Fiedler, Dr. Ulrike
      Rudolph, Prof. Dr. Rainer

<120> Design von Beta-Faltblatt-Proteinen mit spezifischen
      Bindungseigenschaften

<130> P20600

<140>
<141>

<150> DE 199 32 688.6
<151> 1999-07-13

<160> 22

<170> PatentIn Ver. 2.1

<210> 1
<211> 45
<212> DNA
<213> Künstliche Sequenz

<220>
<223> Beschreibung der künstlichen
      Sequenz: Oligonukleotid

<400> 1
cgcgcgcgtc tcacaaagat acatgccatg actcgcggcc cagcc
45

<210> 2
<211> 50
<212> DNA
<213> Künstliche Sequenz

<220>
<223> Beschreibung der künstlichen
      Sequenz: Oligonukleotid

<400> 2
gccgcaggaa gtactggtga ccctggtagt tggggcgctc atacagcatc
50

<210> 3
<211> 41
<212> DNA
<213> Künstliche Sequenz

<220>
<223> Beschreibung der künstlichen
      Sequenz: Oligonukleotid

<400> 3
ccatcagccc catcagcgaa ctttgccgca ggaagtactg g
41

<210> 4
<211> 48
<212> DNA
<213> Künstliche Sequenz

<220>
<223> Beschreibung der künstlichen
      Sequenz: Oligonukleotid

<400> 4
gagtcattct gcggccgcat aaaaatccat cacccgtctt aaagaacc
48

<210> 5
<211> 59
<212> DNA
<213> Künstliche Sequenz

<220>
<223> Beschreibung der künstlichen
      Sequenz: Oligonukleotid

<400> 5
gcggcccagc cggccgctgc tggatgctgt atgagcgccc caactaccag ggtcaccag
59

<210> 6
<211> 55
<212> DNA
<213> Künstliche Sequenz

<220>
<223> Beschreibung der künstlichen
      Sequenz: Oligonukleotid

<400> 6
catgccatga ctcgcggccc agccggccat ggggaagatc actttttacg aggac
55

<210> 7
<211> 26
<212> DNA
<213> Künstliche Sequenz

<220>
<223> Beschreibung der künstlichen
      Sequenz: Oligonukleotid

<400> 7
ccatgattac gccaagcttt ggagcc
26

<210> 8
<211> 21
<212> DNA
<213> Künstliche Sequenz

<220>
<223> Beschreibung der künstlichen
      Sequenz: Oligonukleotid

<400> 8
ctgaaagtgc cggtgtgttg c
21

<210> 9
<211> 176
<212> DNA
<213> Bos sp.

<400> 9
ggcccagccg gccatgggga ggatcaagtt taaagaggac cggggcttcc agggccacta
60
ttacagttgc aatagcgact gccccaacct gcagccctat ttcagccgct gtaactccat
120
cagggtgctg agcggctgct ggatgctgta tgagcgcccc aactaccagg gtcacc
176

<210> 10
<211> 176
<212> DNA
<213> Bos sp.

<400> 10
ggcccagccg gccatgggga agatcacttt ttacgaggac cggggcttcc agggccactg
60
ctacgagtgc agcagcgact gccccaacct gcagccctat ttcagccgct gtaactccat
120
ccgcgtggac agcggctgct ggatgctgta tgagcgcccc aactaccagg gccacc
176

<210> 11
<211> 54
<212> DNA
<213> Künstliche Sequenz

<220>
<223> Beschreibung der künstlichen
      Sequenz: Oligonukleotid

<400> 11
ccccatggcc ggctgggccg cgagtcatgg catgtatctt tgtgagacgc gcgcg
54

<210> 12
<211> 36
<212> DNA
<213> Künstliche Sequenz

<220>
<223> Beschreibung der künstlichen
      Sequenz: Oligonukleotid

<400> 12
ggccatgggg nnkatcnnkt ttnnkgagga ccgggg
36

<210> 13
<211> 24
<212> DNA
<213> Künstliche Sequenz

<220>
<223> Beschreibung der künstlichen
      Sequenz: Oligonukleotid

<400> 13
gtggccctgg aagccccggt cctc
24

<210> 14
<211> 44
<212> DNA
<213> Künstliche Sequenz

<220>
<223> Beschreibung der künstlichen
      Sequenz: Oligonukleotid

<400> 14
cttccagggc cacnnktacn nktgcnnkag cgactgcccc aacc
44

<210> 15
<211> 20
<212> DNA
<213> Künstliche Sequenz

<220>
<223> Beschreibung der künstlichen
      Sequenz: Oligonukleotid

<400> 15
tgcagcccta tttcagccgc
20

<210> 16
<211> 47
<212> DNA
<213> Künstliche Sequenz

<220>
<223> Beschreibung der künstlichen
      Sequenz: Oligonukleotid

<400> 16
gatggagtta cagcggctga aatagggctg caggttgggg cagtcgc
47

<210> 17
<211> 45
<212> DNA
<213> Künstliche Sequenz

<220>
<223> Beschreibung der künstlichen
      Sequenz: Oligonukleotid

<400> 17
tgtaactcca tcnnkgtgnn kagcggctgc tggatgctgt atgag
45

<210> 18
<211> 37
<212> DNA
<213> Künstliche Sequenz

<220>
<223> Beschreibung der künstlichen
      Sequenz: Oligonukleotid

<400> 18
cgccccaact accagggtca ccagtacttc ctgcggc
37

<210> 19
<211> 198
<212> PRT
<213> Bos sp.

<400> 19
Ala Ala Gln Pro Ala Met Gly Arg Ile Lys Phe Lys Glu Asp Arg Gly
1               5                   10                  15

Phe Gln Gly His Tyr Tyr Ser Cys Asn Ser Asp Cys Pro Asn Leu Gln
            20                  25                  30

Pro Tyr Phe Ser Arg Cys Asn Ser Ile Arg Val Leu Ser Gly Cys Trp
        35                  40                  45

Met Leu Tyr Glu Arg Pro Asn Tyr Gln Gly His Gln Tyr Phe Leu Arg
    50                  55                  60

Arg Gly Asp Tyr Pro Asp Tyr Gln Gln Trp Met Gly Phe Asn Asp Ser
65                  70                  75                  80

Ile Arg Ser Cys Arg Leu Ile Pro Gln His Thr Gly Thr Phe Arg Met
                85                  90                  95

Arg Ile Tyr Glu Arg Asp Asp Phe Arg Gly Gln Met Ser Glu Ile Thr
            100                 105                 110

Asp Asp Cys Pro Ser Leu Gln Asp Arg Phe His Leu Thr Glu Val His
        115                 120                 125

Ser Leu Asn Val Leu Glu Gly Ser Trp Val Leu Tyr Glu Met Pro Ser
    130                 135                 140

Tyr Arg Gly Arg Gln Tyr Leu Leu Arg Pro Gly Glu Tyr Arg Arg Tyr
145                 150                 155                 160

Leu Asp Trp Gly Ala Met Asn Ala Lys Val Gly Ser Leu Arg Arg Val
                165                 170                 175

Met Asp Phe Tyr Ala Ala Ala Gly Ala Pro Val Pro Tyr Pro Asp Pro
            180                 185                 190

Leu Glu Pro Arg Ala Ala
        195


<210> 20
<211> 198
<212> PRT
<213> Bos sp.

<400> 20
Ala Ala Gln Pro Ala Met Gly Lys Ile Thr Phe Tyr Glu Asp Arg Gly
1               5                   10                  15

Phe Gln Gly His Cys Tyr Glu Cys Ser Ser Asp Cys Pro Asn Leu Gln
            20                  25                  30

Pro Tyr Phe Ser Arg Cys Asn Ser Ile Arg Val Asp Ser Gly Cys Trp
        35                  40                  45

Met Leu Tyr Glu Arg Pro Asn Tyr Gln Gly His Gln Tyr Phe Leu Arg
    50                  55                  60

Arg Gly Asp Tyr Pro Asp Tyr Gln Gln Trp Met Gly Phe Asn Asp Ser
65                  70                  75                  80

Ile Arg Ser Cys Arg Leu Ile Pro Gln His Thr Gly Thr Phe Arg Met
                85                  90                  95

Arg Ile Tyr Glu Arg Asp Asp Phe Arg Gly Gln Met Ser Glu Ile Thr
            100                 105                 110

Asp Asp Cys Pro Ser Leu Gln Asp Arg Phe His Leu Thr Glu Val His
        115                 120                 125

Ser Leu Asn Val Leu Glu Gly Ser Trp Val Leu Tyr Glu Met Pro Ser
    130                 135                 140

Tyr Arg Gly Arg Gln Tyr Leu Leu Arg Pro Gly Glu Tyr Arg Arg Tyr
145                 150                 155                 160

Leu Asp Trp Gly Ala Met Asn Ala Lys Val Gly Ser Leu Arg Arg Val
                165                 170                 175

Met Asp Phe Tyr Ala Ala Ala Gly Ala Pro Val Pro Tyr Pro Asp Pro
            180                 185                 190

Leu Glu Pro Arg Ala Ala
        195

<210> 21
<211> 197
<212> PRT
<213> Bos sp.

<400> 21
Met Gly Arg Ile Lys Phe Lys Glu Asp Arg Gly Phe Gln Gly His Tyr
1               5                   10                  15

Tyr Ser Cys Asn Ser Asp Cys Pro Asn Leu Gln Pro Tyr Phe Ser Arg
            20                  25                  30

Cys Asn Ser Ile Arg Val Leu Ser Gly Cys Trp Met Leu Tyr Glu Arg
        35                  40                  45

Pro Asn Tyr Gln Gly His Gln Tyr Phe Leu Arg Arg Gly Asp Tyr Pro

50                  55                  60

Asp Tyr Gln Gln Trp Met Gly Phe Asn Asp Ser Ile Arg Ser Cys Arg
65                  70                  75                  80

Leu Ile Pro Gln His Thr Gly Thr Phe Arg Met Arg Ile Tyr Glu Arg
                85                  90                  95

Asp Asp Phe Arg Gly Gln Met Ser Glu Ile Thr Asp Asp Cys Pro Ser
            100                 105                 110

Leu Gln Asp Arg Phe His Leu Thr Glu Val His Ser Leu Asn Val Leu
        115                 120                 125

Glu Gly Ser Trp Val Leu Tyr Glu Met Pro Ser Tyr Arg Gly Arg Gln
    130                 135                 140

Tyr Leu Leu Arg Pro Gly Glu Tyr Arg Arg Tyr Leu Asp Trp Gly Ala
145                 150                 155                 160

Met Asn Ala Lys Val Gly Ser Leu Arg Arg Val Met Asp Phe Tyr Ser
                165                 170                 175

Asp Pro Asn Ser Ser Ser Val Asp Lys Leu Ala Ala Ala Leu Glu His
            180                 185                 190

His His His His His
        195

<210> 22
<211> 197
<212> PRT
<213> Bos sp.

<400> 22
Met Gly Lys Ile Thr Phe Tyr Glu Asp Arg Gly Phe Gln Gly His Cys
1               5                   10                  15

Tyr Glu Cys Ser Ser Asp Cys Pro Asn Leu Gln Pro Tyr Phe Ser Arg
            20                  25                  30

Cys Asn Ser Ile Arg Val Asp Ser Gly Cys Trp Met Leu Tyr Glu Arg
        35                  40                  45

Pro Asn Tyr Gln Gly His Gln Tyr Phe Leu Arg Arg Gly Asp Tyr Pro
    50                  55                  60

Asp Tyr Gln Gln Trp Met Gly Phe Asn Asp Ser Ile Arg Ser Cys Arg
65                  70                  75                  80

Leu Ile Pro Gln His Thr Gly Thr Phe Arg Met Arg Ile Tyr Glu Arg

85                  90                  95

Asp Asp Phe Arg Gly Gln Met Ser Glu Ile Thr Asp Asp Cys Pro Ser
            100                 105                 110

Leu Gln Asp Arg Phe His Leu Thr Glu Val His Ser Leu Asn Val Leu
        115                 120                 125

Glu Gly Ser Trp Val Leu Tyr Glu Met Pro Ser Tyr Arg Gly Arg Gln
    130                 135                 140

Tyr Leu Leu Arg Pro Gly Glu Tyr Arg Arg Tyr Leu Asp Trp Gly Ala
145                 150                 155                 160

Met Asn Ala Lys Val Gly Ser Leu Arg Arg Val Met Asp Phe Tyr Ser
                165                 170                 175

Asp Pro Asn Ser Ser Ser Val Asp Lys Leu Ala Ala Ala Leu Glu His
            180                 185                 190

His His His His His
        195

SEQUENZPROTOKOLL

<110> Scil Proteins GmbH

<120> Design von Beta-Faltblatt-Proteinen mit spezifischen
      Bindungseigenschaften

<130> P20600

<140> EP 06118519.5
<141> 2000-07-13

<150> DE 199 32 688.6
<151> 1999-07-13

<160> 22

<170> PatentIn Ver. 2.1

<210> 1
<211> 45
<212> DNA
<213> Künstliche Sequenz

<220>
<223> Beschreibung der künstlichen
      Sequenz: Oligonukleotid

<400> 1
cgcgcgcgtc tcacaaagat acatgccatg actcgcggcc cagcc                45

<210> 2
<211> 50
<212> DNA
<213> Künstliche Sequenz

<220>
<223> Beschreibung der künstlichen
      Sequenz: Oligonukleotid

<400> 2
gccgcaggaa gtactggtga ccctggtagt tggggcgctc atacagcatc           50

<210> 3
<211> 41
<212> DNA
<213> Künstliche Sequenz

<220>
<223> Beschreibung der künstlichen
      Sequenz: Oligonukleotid

<400> 3
ccatcagccc catcagcgaa ctttgccgca ggaagtactg g                    41

<210> 4
<211> 48
<212> DNA
<213> Künstliche Sequenz

<220>
<223> Beschreibung der künstlichen
      Sequenz: Oligonukleotid

<400> 4
gagtcattct gcggccgcat aaaaatccat cacccgtctt aaagaacc 48

<210> 5
<211> 59
<212> DNA
<213> Künstliche Sequenz

<220>
<223> Beschreibung der künstlichen
      Sequenz: Oligonukleotid

<400> 5
gcggcccagc cggccgctgc tggatgctgt atgagcgccc caactaccag ggtcaccag 59

<210> 6
<211> 55
<212> DNA
<213> Künstliche Sequenz

<220>
<223> Beschreibung der künstlichen
      Sequenz: Oligonukleotid

<400> 6
catgccatga ctcgcggccc agccggccat ggggaagatc actttttacg aggac 55

<210> 7
<211> 26
<212> DNA
<213> Künstliche Sequenz

<220>
<223> Beschreibung der künstlichen
      Sequenz: Oligonukleotid

<400> 7
ccatgattac gccaagcttt ggagcc 26

<210> 8
<211> 21
<212> DNA
<213> Künstliche Sequenz

<220>
<223> Beschreibung der künstlichen
      Sequenz: Oligonukleotid

<400> 8
ctgaaagtgc cggtgtgttg c 21

<210> 9
<211> 176
<212> DNA
<213> Bos sp.

<400> 9
ggcccagccg gccatgggga ggatcaagtt taaagaggac cggggcttcc agggccacta 60
ttacagttgc aatagcgact gccccaacct gcagccctat ttcagccgct gtaactccat 120
cagggtgctg agcggctgct ggatgctgta tgagcgcccc aactaccagg gtcacc 176

<210> 10

<211> 176
<212> DNA
<213> Bos sp.

<400> 10
ggcccagccg gccatgggga agatcacttt ttacgaggac cggggcttcc agggccactg 60
ctacgagtgc agcagcgact gccccaacct gcagccctat ttcagccgct gtaactccat 120
ccgcgtggac agcggctgct ggatgctgta tgagcgcccc aactaccagg gccacc 176

<210> 11
<211> 55
<212> DNA
<213> Künstliche Sequenz

<220>
<223> Beschreibung der künstlichen
      Sequenz: Oligonukleotid

<400> 11
ccccatggcc ggctgggccg cgagtcatgg catgtatctt tgtgagacgc gcgcg 55

<210> 12
<211> 36
<212> DNA
<213> Künstliche Sequenz

<220>
<223> Beschreibung der künstlichen
      Sequenz: Oligonukleotid

<220>
<221> misc_feature
<222> (11)..(12)
<223> n is a, c, g, or t

<220>
<221> misc_feature
<222> (17)..(18)
<223> n is a, c, g, or t

<220>
<221> misc_feature
<222> (23)..(24)
<223> n is a, c, g, or t

<400> 12
ggccatgggg nnkatcnnkt ttnnkgagga ccgggg 36

<210> 13
<211> 24
<212> DNA
<213> Künstliche Sequenz

<220>
<223> Beschreibung der künstlichen
      Sequenz: Oligonukleotid

<400> 13
gtggccctgg aagccccggt cctc 24

<210> 14
<211> 44
<212> DNA

<213> Künstliche Sequenz

<220>
<223> Beschreibung der künstlichen
      Sequenz: Oligonukleotid

<220>
<221>  misc_feature
<222>  (14)..(15)
<223>  n is a, c, g, or t

<220>
<221>  misc_feature
<222>  (20)..(21)
<223>  n is a, c, g, or t

<220>
<221>  misc_feature
<222>  (26)..(27)
<223>  n is a, c, g, or t

<400> 14
cttccagggc cacnnktacn nktgcnnkag cgactgcccc aacc                44

<210> 15
<211> 20
<212> DNA
<213> Künstliche Sequenz

<220>
<223> Beschreibung der künstlichen
      Sequenz: Oligonukleotid

<400> 15
tgcagcccta tttcagccgc                                           20

<210> 16
<211> 47
<212> DNA
<213> Künstliche Sequenz

<220>
<223> Beschreibung der künstlichen
      Sequenz: Oligonukleotid

<400> 16
gatggagtta cagcggctga aatagggctg caggttgggg cagtcgc             47

<210> 17
<211> 45
<212> DNA
<213> Künstliche Sequenz

<220>
<223> Beschreibung der künstlichen
      Sequenz: Oligonukleotid

<220>
<221>  misc_feature
<222>  (13)..(14)
<223>  n is a, c, g, or t

<220>

<221>  misc_feature
<222>  (19)..(20)
<223>  n is a, c, g, or t

<400> 17
tgtaactcca tcnnkgtgnn kagcggctgc tggatgctgt atgag                45

<210> 18
<211> 37
<212> DNA
<213> Künstliche Sequenz

<220>
<223> Beschreibung der künstlichen
      Sequenz: Oligonukleotid

<400> 18
cgccccaact accagggtca ccagtacttc ctgcggc                         37

<210> 19
<211> 198
<212> PRT
<213> Bos sp.

<400> 19
Ala Ala Gln Pro Ala Met Gly Arg Ile Lys Phe Lys Glu Asp Arg Gly
1               5                   10                  15

Phe Gln Gly His Tyr Tyr Ser Cys Asn Ser Asp Cys Pro Asn Leu Gln
            20                  25                  30

Pro Tyr Phe Ser Arg Cys Asn Ser Ile Arg Val Leu Ser Gly Cys Trp
        35                  40                  45

Met Leu Tyr Glu Arg Pro Asn Tyr Gln Gly His Gln Tyr Phe Leu Arg
    50                  55                  60

Arg Gly Asp Tyr Pro Asp Tyr Gln Gln Trp Met Gly Phe Asn Asp Ser
65                  70                  75                  80

Ile Arg Ser Cys Arg Leu Ile Pro Gln His Thr Gly Thr Phe Arg Met
                85                  90                  95

Arg Ile Tyr Glu Arg Asp Asp Phe Arg Gly Gln Met Ser Glu Ile Thr
            100                 105                 110

Asp Asp Cys Pro Ser Leu Gln Asp Arg Phe His Leu Thr Glu Val His
        115                 120                 125

Ser Leu Asn Val Leu Glu Gly Ser Trp Val Leu Tyr Glu Met Pro Ser
    130                 135                 140

Tyr Arg Gly Arg Gln Tyr Leu Leu Arg Pro Gly Glu Tyr Arg Arg Tyr
145                 150                 155                 160

Leu Asp Trp Gly Ala Met Asn Ala Lys Val Gly Ser Leu Arg Arg Val
                165                 170                 175

Met Asp Phe Tyr Ala Ala Ala Gly Ala Pro Val Pro Tyr Pro Asp Pro
            180                 185                 190

Leu Glu Pro Arg Ala Ala
        195

<210> 20
<211> 198
<212> PRT
<213> Bos sp.

<400> 20
Ala Ala Gln Pro Ala Met Gly Lys Ile Thr Phe Tyr Glu Asp Arg Gly
1               5                   10                  15

Phe Gln Gly His Cys Tyr Glu Cys Ser Ser Asp Cys Pro Asn Leu Gln
            20                  25                  30

Pro Tyr Phe Ser Arg Cys Asn Ser Ile Arg Val Asp Ser Gly Cys Trp
        35                  40                  45

Met Leu Tyr Glu Arg Pro Asn Tyr Gln Gly His Gln Tyr Phe Leu Arg
    50                  55                  60

Arg Gly Asp Tyr Pro Asp Tyr Gln Gln Trp Met Gly Phe Asn Asp Ser
65                  70                  75                  80

Ile Arg Ser Cys Arg Leu Ile Pro Gln His Thr Gly Thr Phe Arg Met
                85                  90                  95

Arg Ile Tyr Glu Arg Asp Asp Phe Arg Gly Gln Met Ser Glu Ile Thr
            100                 105                 110

Asp Asp Cys Pro Ser Leu Gln Asp Arg Phe His Leu Thr Glu Val His
        115                 120                 125

Ser Leu Asn Val Leu Glu Gly Ser Trp Val Leu Tyr Glu Met Pro Ser
    130                 135                 140

Tyr Arg Gly Arg Gln Tyr Leu Leu Arg Pro Gly Glu Tyr Arg Arg Tyr
145                 150                 155                 160

Leu Asp Trp Gly Ala Met Asn Ala Lys Val Gly Ser Leu Arg Arg Val
                165                 170                 175

Met Asp Phe Tyr Ala Ala Ala Gly Ala Pro Val Pro Tyr Pro Asp Pro
            180                 185                 190

Leu Glu Pro Arg Ala Ala
        195

<210> 21
<211> 197
<212> PRT
<213> Bos sp.

<400> 21
Met Gly Arg Ile Lys Phe Lys Glu Asp Arg Gly Phe Gln Gly His Tyr
1               5                   10                  15

Tyr Ser Cys Asn Ser Asp Cys Pro Asn Leu Gln Pro Tyr Phe Ser Arg
            20                  25                  30

Cys Asn Ser Ile Arg Val Leu Ser Gly Cys Trp Met Leu Tyr Glu Arg
        35                  40                  45

Pro Asn Tyr Gln Gly His Gln Tyr Phe Leu Arg Arg Gly Asp Tyr Pro
    50                  55                  60

Asp Tyr Gln Gln Trp Met Gly Phe Asn Asp Ser Ile Arg Ser Cys Arg
65                  70                  75                  80

Leu Ile Pro Gln His Thr Gly Thr Phe Arg Met Arg Ile Tyr Glu Arg
                85                  90                  95

Asp Asp Phe Arg Gly Gln Met Ser Glu Ile Thr Asp Asp Cys Pro Ser
            100                 105                 110

Leu Gln Asp Arg Phe His Leu Thr Glu Val His Ser Leu Asn Val Leu
        115                 120                 125

Glu Gly Ser Trp Val Leu Tyr Glu Met Pro Ser Tyr Arg Gly Arg Gln
    130                 135                 140

Tyr Leu Leu Arg Pro Gly Glu Tyr Arg Arg Tyr Leu Asp Trp Gly Ala
145                 150                 155                 160

Met Asn Ala Lys Val Gly Ser Leu Arg Arg Val Met Asp Phe Tyr Ser
                165                 170                 175

Asp Pro Asn Ser Ser Ser Val Asp Lys Leu Ala Ala Ala Leu Glu His
            180                 185                 190

His His His His His
        195

<210> 22
<211> 197
<212> PRT
<213> Bos sp.

<400> 22
Met Gly Lys Ile Thr Phe Tyr Glu Asp Arg Gly Phe Gln Gly His Cys
1               5                   10                  15

Tyr Glu Cys Ser Ser Asp Cys Pro Asn Leu Gln Pro Tyr Phe Ser Arg
            20                  25                  30

Cys Asn Ser Ile Arg Val Asp Ser Gly Cys Trp Met Leu Tyr Glu Arg
        35                  40                  45

Pro Asn Tyr Gln Gly His Gln Tyr Phe Leu Arg Arg Gly Asp Tyr Pro
    50                  55                  60

Asp Tyr Gln Gln Trp Met Gly Phe Asn Asp Ser Ile Arg Ser Cys Arg
65                  70                  75                  80

Leu Ile Pro Gln His Thr Gly Thr Phe Arg Met Arg Ile Tyr Glu Arg
                85                  90                  95

Asp Asp Phe Arg Gly Gln Met Ser Glu Ile Thr Asp Asp Cys Pro Ser
            100                 105                 110

Leu Gln Asp Arg Phe His Leu Thr Glu Val His Ser Leu Asn Val Leu
        115                 120                 125

Glu Gly Ser Trp Val Leu Tyr Glu Met Pro Ser Tyr Arg Gly Arg Gln

    130                 135                 140

Tyr Leu Leu Arg Pro Gly Glu Tyr Arg Arg Tyr Leu Asp Trp Gly Ala
145                 150                 155                 160

Met Asn Ala Lys Val Gly Ser Leu Arg Arg Val Met Asp Phe Tyr Ser
                165                 170                 175

Asp Pro Asn Ser Ser Ser Val Asp Lys Leu Ala Ala Ala Leu Glu His
            180                 185                 190

His His His His His
        195

## Beschreibung

Die vorliegende Erfindung betrifft neuartige Beta-Faltblatt-Proteine mit neuen oder veränderten spezifischen Bindungseigenschaften oder einer neuen oder veränderten katalytischen Aktivität oder neuen oder veränderten Fluoreszenzeigenschaften sowie Verfahren zur Herstellung derartig modifizierter Proteine.

Antikörper und deren Derivate werden in vielen Bereichen der humanen und veterinären Therapie, Diagnostik sowie beim Monitoring eingesetzt. Ein Problem bei der Nutzung der natürlich vorkommenden Antikörper ist deren Herstellung. Nach wie vor erfolgt die Produktion der Antikörper im tierischen Zellkultursystem, ein Verfahren, das sehr kostenintensiv ist. Für einige Anwendungen, wie z. B. die Herstellung von Fusionsproteinen oder ein Einsatz in der Therapie, der eine schnelle Blut-clearance und gute Gewebepenetration notwendig macht, stellt die Größe der natürlich vorkommenden Antikörpermoleküle ein weiteres Problem dar (Colcher et al., 1998). Rekombinante Antikörpermoleküle, wie scFv's (Bird et al., 1988), Miniantikörper (Pack und Plückthun, 1992) oder bispezifische Antikörper (Holliger und Winter, 1993) sind größtenteils nur noch aus den antigenbindenden Domänen der Antikörper (VH und VL) aufgebaut. Aufgrund ihrer erheblich verringerten Größe zeigen sie eine verbesserte Gewebepenetration und sind auch für Fusionen mit anderen Proteinen besser als vollständige Antikörper geeignet. Gegenüber diesen sind rekombinante Antikörperfragmente allerdings oft instabier, haben geringere Affinitäten, und sind aufgrund der auszubildenden Disulfidbrücken schwer rekombinant herzustellen. Methoden zur Stabilisierung und verbesserten Affinität der rekombinanten Antikörperfragmente beinhalten u.a. die Testung verschiedener Linkerpeptide und die Einführung von Disulfidverbrückungen (Glockshuber et al., 1990, Cumber et al., 1992, Brinkmann, 1997).

Die Sequenz und Länge der Linkerpeptide kann sowohl die Stabilität gegenüber Proteasen als auch die Affinität der Antikörperfragmente beeinflussen (Pantoliano et al., 1991). Die Einführung von zusätzlichen Disulfidverbrückungen in die konservierten*framework*-Regionen der variablen Domänen kann zu einer erhöhten Resistenz gegenüber Hitze (Young et al., 1995) und denaturierenden Agenzien, sowie zu verbesserten Ausbeuten bei heterologer Expression führen. Generell zeigen viele scFv's jedoch eine geringe Stabilität und neigen bereits bei 37°C zur Aggregation. Die Instabilität kann ebenfalls durch die Verwendung der allgemeinen Fv-Fragment-Klonierungsprimer verursacht werden, durch die neue destabilisierende Mutationen eingeführt werden können. Die Produktion der Antikörperfragmente im bakteriellen System erfolgt meist durch Ausschleusen in den periplasmatischen Raum, wobei auch hier Optimierungen hinsichtlich des Redoxzustandes bzw. der gleichzeitigen Expression von Faltungshelfern möglich sind.

Es ist eine Aufgabe der vorliegenden Erfindung, neue Proteine bereitzustellen, die neue oder veränderte Bindungseigenschaften, beispielsweise antikörperähnliche Eigenschaften, aufweisen, gleichzeitig jedoch die oben beschriebenen Nachteile vollständiger oder rekombinanter Antikörpermoleküle nicht zeigen.

Eine weitere Aufgabe der vorliegenden Erfindung liegt darin, Proteine bereitzustellen, die neue oder veränderte enzymatische bzw. katalytische Eigenschaften zeigen.

Eine weitere Aufgabe der vorliegenden Erfindung liegt in der Schaffung von Verfahren zur Bildung der oben genannten Proteine.

Die oben genannten Aufgaben werden durch ein Protein mit den in Anspruch 1 gekennzeichneten Merkmalen gelöst. Ein Verfahren zur Herstellung der erfindungsgemäßen Proteine ergibt sich aus dem Anspruch 21. Bevorzugte Ausgestaltungen der Erfindung ergeben sich aus den Unteransprüchen und der nachfolgenden Beschreibung.

Durch Veränderung der Oberfläche eines Beta-Faltblatt-Proteins werden neuartige, vorher nicht vorhandene Bindungseigenschaften im Protein erzeugt. Diese Bindungseigenschaften werden durch Mutagenese eines Beta-Faltblatt-Bereiches geschaffen. Die neuartigen Beta-Faltblatt-Proteine sind trotz der de novo Bindungseigenschaften den Ausgangsproteinen in Struktur und Stabilität ähnlich. Ausgangsproteine für das Design der neuartigen Bindungsmoleküle sind Proteine mit einer überwiegenden Beta-Faltblatt-Struktur, wie z. B. das Gamma-Kristallin, ein Strukturprotein der Augenlinse. Basierend auf der Kristallstruktur werden z.B. mittels Computeranalysen Bereiche und Aminosäuren im Beta-Faltblatt der Ausgangsproteine ausgewählt, die oberflächenexponiert und damit dem Lösungsmittel bzw. möglichen Bindungspartnern zugänglich sind. In dem für das Ausgangsprotein kodierenden Gen werden diese Bereiche bzw. Aminosäurepositionen mit gentechnischen Methoden mutagenisiert. Auf DNA-Ebene wird so eine Vielzahl an mutierten Genen (Bank oder Bibliothek), die für die unterschiedlichen Beta-Faltblatt-Protein-Mutanten kodieren, hergestellt. Die Isolierung von Mutanten mit neuartigen, gewünschten Bindungseigenschaften erfolgt mit Hilfe eines geeigneten Selektionssystems, wie z. B. dem *Phage Display*-System. Beim *Phage Display* wird die Gesamtheit der erstellten Protein-Mutanten auf der Oberfläche von Bakteriophagen exponiert (*Phage Display-*Bank)*.* Diese rekombinanten Phagen werden hinsichtlich ihrer Bindung an die gewünschten Zielmoleküle untersucht. Phagen, die auf ihrer Oberfläche Beta-Faltblatt-Mutanten mit einer spezifischen Bindung zum Zielmolekül exponieren, werden durch wiederholtes Screening angereichert und isoliert. Gene, die für bindende Beta-Faltblatt-Mutanten kodieren, werden von den Phagen erhalten und in einem geeigneten Expressionssystem, wie z. B.*E. coli*, exprimiert. Mit dem beschriebenen Verfahren gelingt es überraschenderweise, aus Beta-Faltblatt-Proteinen, welche keinerlei spezifische Bindungseigenschaften aufweisen, spezifischbindende Proteine herzustellen, wobei Mutanten mit der gewünschten Spezifität aus der Bibliothek durch Anwendung eines geeigneten Screeningverfahrens isoliert werden. In Abhängigkeit von den Eigenschaften des Ausgangsproteins haben die mit dem beschriebenen System erstellten Beta-Faltblatt-Mutanten z.B. gegenüber Antikörpern und rekombinanten Antikörperfragmenten Vorteile hinsichtlich der Größe, Stabilität und funktionell aktiver Produktion im heterologen, vorzugsweise bakteriellen System. Diese verbesserten Eigenschaften der neuartigen Beta-Faltblatt-Mutanten ermöglichen es, z.B. Antikörper, rekombinante Antikörperfragmente oder katalytische Antikörper zu ersetzen sowie ganz neue Anwendungsbereiche zu erschließen.

Die erfindungsgemäße Lösung beispielsweise der dargestellten Antikörperproblematik liegt im Design von Proteinen mit jeweils spezifischen Bindungseigenschaften, die eine hohe Stabilität gegenüber geringen pH-Werten, denaturierenden Agenzien und erhöhter Temperatur aufweisen, d. h. die Bedingungen standhalten, bei denen Antikörper instabil sind. Die Schaffung von Proteinen mit Beta-Faltblatt-Struktur mit antikörperähnlichen Bindungseigenschaften ist aber nur ein mögliches Anwendungsgebiet der vorliegenden Erfindung. Weitere Anwendungsmöglichkeiten erschließen sich beispielsweise bei der Schaffung von Beta-Faltblatt-Proteinen mit neuen katalytischen Eigenschaften, z.B. Resistenzeigenschaften und fluoreszierenden Eigenschaften. Ein Beispiel für ein Protein, dessen fluoreszierende Eigenschaften veränderbar sind, ist das GFP. Kleine Proteine mit einer natürlicherweise hohen Stabilität sind für das Design besonders geeignet. Durch Veränderung ihrer Oberfläche wurden erfindungsgemäß, unter Beibehaltung der Stabilität, beispielhaft neue spezifische Bindungseigenschaften im Protein erzeugt.

Erfindungsgemäß wurden als eine mögliche Klasse stabiler Proteine beispielhaft Kristalline ausgewählt. Kristalline, die Strukturproteine der Augenlinsen, unterliegen üblicherweise keinem zellulären "turnover" und weisen demzufolge auch außerordentliche Stabilitätseigenschaften auf (Mandal et al., 1987, Rudolph et al., 1990). Gamma-Kristalline, eine Klasse der Kristalline bei Vertebraten, sind monomere Proteine mit einer Molekularmasse von etwa 22 kDa. Das Hauptstrukturmotiv der Gamma-Kristalline ist das antiparallele Beta-Faltblatt (Hazes und Hol, 1992, Richardson et al., 1992, Hemmingsen et al., 1994). Gamma-Kristalline bestehen aus zwei sehr ähnlichen globulären Domänen, einer N- und einer C-terminalen Domäne, die miteinander durch ein V-förmiges Linkerpeptid verbunden sind. Das für Gamma-Kristalline charakteristische Faltungsmuster ("Greek-Key"-Motiv, Slingsby, 1985, Wistow und Piatigorsky, 1988) ist höchstwahrscheinlich die Ursache für die beträchtliche Thermostabilität sowie die Stabilität gegenüber Denaturierungsmitteln (Mandal et al., 1987). Das Gamma-II-Kristallin aus Kälberaugen ist ein 21 kDa-Protein mit für seine Größe ungewöhnlich vielen (7) Cysteinen, die unter physiologischen Bedingungen reduziert vorliegen.

Gamma-II-Kristallin weist im normal gefalteten Zustand keinerlei Bindungseigenschaften auf. Die erfindungsgemäße Veränderung (Mutagenese) einer ausgewählten, lösungsmittelexponierten Region dieses Proteins, die aus dem Strukturmotiv des Beta-Faltblattes besteht, führte überraschenderweise zur Veränderung der Oberflächenstruktur und dem Ladungsmuster des Proteins und somit zur Herstellung neuer Bindungseigenschaften. Dabei wurden nur Bereiche oder Aminosäurepositionen ausgewählt, die nicht maßgeblich an der Strukturerhaltung des Proteins beteiligt sind. Die Mutagenese eines kleinen Beta-Faltblatt-Proteins (Riddle et al., 1997) hat gezeigt, daß Proteine trotz erheblicher Sequenzänderungen zu einem hohen Prozentsatz korrekt die native Struktur des Beta-Faltblatts ausbilden können.

Ansätze zur Mutagenese von bestimmten Proteinbereichen mit dem Ziel der Isolierung von Molekülen mit verbesserten oder neuen Bindungseigenschaften existierten bereits für rekombinante Antikörperfragmente (Nissim et al., 1994, de Kruif et al., 1995), für Proteine mit bereits vorhandenen Bindungseigenschaften (Rezeptoren, Inhibitorproteine, DNA-bindende Proteine) sowie für *peptide libraries* (Cortese et al., 1995, Haaparanta und Huse 1995, McConell et al., 1996). Im Falle der Antikörper werden nur die antigenbindenden Domänen, die als loop-Regionen vorliegen, mutagenisiert. Dies ist ebenfalls für die meisten anderen Proteine, wie z. B. Tendamistat (McConell und Hoess, 1995) oder Cytochrom b₅₆₂ (Ku und Schultz, 1995), der Fall. Auch hier werden loop-Regionen mutagenisiert. Beispiele für Mutagenesen in alpha-Helices sind die Z-Domäne vom Protein A (Nord et al., 1997) und die Zinkfingerdomäne CP-1 (Choo und Klug, 1995). Bei den bisherigen Mutagenesen wurde lediglich die Spezifität der Bindung verändert, wobei immer von Proteinen mit bereits vorhandenen Bindungseigenschaften ausgegangen wurde. In keinem Fall wurde ein Protein ohne Bindungseigenschaften verwendet, noch wurde ein Beta-Faltblatt-Strukturmotiv gezielt verändert. Bei dem hier beschriebenen Verfahren wurde erstmals an einem Protein ohne jegliche Bindungseigenschaften in dem starren Bereich des Beta-Faltblatts eine gezielte Mutagenese durchgeführt. Dadurch wurde unerwarteterweise ein Protein mit erheblicher Stabilität und mit spezifischen Bindungseigenschaften, vergleichbar mit Antikörpermolekülen, erzeugt.

Als geeignetes System für die Isolierung mutagenisierter Beta-Faltblatt-Proteine mit neu entstandenen Bindungseigenschaften dient das *Phage Display*-System. Das System ermöglicht ein sehr effizientes Screening eines großen Repertoires an Proteinvarianten auf spezifische Bindungseigenschaften (Smith, 1985). Dabei wird jeweils eine Proteinvariante auf der Oberfläche eines filamentösen Phagen dargestellt und kann mit den Zielmolekülen, die an einer Festphase immobilisiert sind, in Wechselwirkung treten. An das Zielmolekül bindende Proteine können durch Elution der Phagen erhalten werden. Nach Isolierung der Phagen-DNA kann die DNA-Sequenz der spezifisch bindenden Proteinvarianten bestimmt werden. Neben dem *Phage Display-*System können auch andere Selektions-Systeme, wie z. B. das Bakterien-*Display* (Stahl und Uhlen, 1997) oder das Ribosomen-*Display* (Hanes et al., 1997), Anwendung finden.

Mit der beschriebenen Erfindung gelingt es überraschenderweise, beispielsweise das sehr stabile Beta-Faltblatt-Protein Gamma-II-Kristallin durch gezielte ortsspezifische Mutagenese im Beta-Faltblatt an der Oberfläche so zu verändern, daß aus dem nicht-bindenden Protein ein Protein mit spezifischen Bindungseigenschaften entsteht. Durch eine Randomisierung von acht Aminosäurepositionen erfolgt damit erstmals eine Mutagenese in einem Gerüstmolekül innerhalb ehes relativ starren Bereich des Proteins. Somit wird aus dem Beta-Faltblatt-Protein Gamma-II-Kristallin eine bezüglich ihrer spezifischen Bindungseigenschaften "antikörperähnliche" Proteinspezies hergestellt. Gamma-II-Kristallin oder andere kleine, stabile Beta-Faltblatt-Proteine können mit dem beschriebenen Verfahren generell als neuartige Gerüstmoleküle für das Design neuartiger Bindungseigenschaften verwendet werden. Die modellierten Beta-Faltblatt-Proteine können z.B. rekombinante Antikörper in verschiedenen Applikationen ersetzen. Aufgrund ihrer relativ geringen Größe (20 kDa) eignen sie sich als Fusionspartner für andere funktionelle Proteine (Herstellung multifunktioneller Proteine). Weitere Einsatzmöglichkeiten liegen in der Gentherapie, wo sie als Module für das zellspezifische Targeting der gentherapeutischen Vektoren Verwendung finden können, und in der intrazellulären Immunisierung. Weiterhin können Beta-Faltblatt-Mutanten mit katalytischen Eigenschaften in Anwendungsbereichen der Enzyme eingesetzt werden. Die Stabilität der neuartigen Bindungsproteine ermöglicht zudem Anwendungen, die derzeit mit rekombinanten Antikörpern nicht durchführbar sind, z. B. in der humanmedizinischen und veterinärmedizinschen Diagnostik und Therapie, der Biosensorik und der Bioseparation. Weitere Anwendungsgebiete sind allgemein die Pharma- und Kosmetikindustrie sowie die Schadstoffanalyse und -beseitigung.

Nachfolgend werden einige bevorzugte Ausgestaltungsformen der Erfindung beschrieben.

Die erfindungsgemäß zur Mutagenese ausgewählten Proteine mit Beta-Faltblatt-Struktur weisen entweder keine Bindungseigenschaften oder keine katalytische bzw. enzymatische Aktivität oder Fluoreszenzeigenschaften auf oder eine solche Aktivität, Fluoreszenzeigenschaften oder solche Bindungseigenschaften, daß eine Veränderung, insbesondere Verbesserung, wünschenswert erscheint.

Proteine mit Beta-Faltblatt-Struktur sind bekannt. Ein Beispiel für eine Proteinklasse mit Beta-Faltblatt sind die Kristalline, insbesondere die Alpha-, Beta- und Gammakristalline. Grundsätzlich einsetzbar sind Kristalline aus allen Tierarten, beispielsweise aus Wirbeltieren, Nagern, Vögeln und Fischen. Weitere Beispiele für Proteine mit Beta-Faltblatt-Struktur, die erfindungsgemäß mutagenisierbar sind, sind: Spheruline, Hitzestreßproteine, Kälteschockproteine, Beta-Helix-Proteine, Lipocaline, Certine oder Transkriptionsfaktoren, Fibronectine, GFP, NGF, Tendamistat oder Lysozym. Erfindungsgemäß mutagenisiert werden z.B. einzelne Untereinheiten oder Domänen dieser Proteine, bspw. der Kristalline, mit Beta-Faltblatt-Struktur.

Unter den Kristallinen ist besonders bevorzugt das Gamma-Kristallin zu nennen, von dem erfindungsgemäß beispielhaft gezeigt werden konnte, daß dessen Beta-Faltblatt-Struktur so abgeändert, d.h. mutagenisiert werden kann, daß neue spezifische Bindungseigenschaften oder neue katalytische Aktivitäten entstehen, die z.B. einem Antikörpermolekül vergleichbar sind. Ein Beispiel für ein Gamma-Kristallin ist das Gamma-II-Kristallin.

Beispiele für Beta-Helix-Proteine finden sich u.a. in Jenkins J. et al., J. Struct. Biol. 1998, 122 (1-2): 236-46, Pickersgill, R. et al., J. Biol. Chem. 1998, 273 (38), 24600-4 und Raetz C.R. et al., Science 1995, 270 (5238), 997-1000.

Die Beta-Faltblatt-Struktur ist dadurch definiert, daß sie im wesentlichen plattenförmig und fast völlig gestreckt vorliegt. Im Gegensatz zu alpha-Helices, die aus einem kontinuierlichen Teil der Polypeptidkette gebildet werden, können Beta-Faltblätter aus verschiedenen Regionen der Polypeptidkette aufgebaut sein. Dadurch können in der Primärstruktur weiter auseinanderliegende Bereiche in unmittelbare Nähe gelangen. Ein Beta-Strang ist normalerweise 5-10 Aminosäuren lang und liegt fast völlig gestreckt vor. Die Beta-Stränge liegen so eng beieinander, daß sich Wasserstoffbrücken zwischen der C=O-Gruppe des einen und der NH-Gruppe des anderen Strangs bzw. umgekehrt ausbilden. Beta-Faltblätter können aus mehreren Strängen aufgebaut sein und haben eine plattenförmige Struktur. Dabei liegt das C-alpha-Atom immer abwechselnd über oder unter der plattenförmigen Ebene. Die Seitenketten der Aminosäuren folgen diesem Muster und sind somit alternativ einmal nach oben und einmal nach unten gerichtet. Je nach Orientierung der Beta-Stränge unterscheidet man parallele und antiparallele Faltblätter. Erfindungsgemäß können beide mutagenisiert und zur Herstellung der beanspruchten Proteine eingesetzt werden.

Zur Mutagenese der Beta-Faltblatt-Struktur werden solche Beta-Faltblatt-Regionen im Protein ausgewählt, die oberflächennah sind. Oberflächenexponierte Aminosäuren können anhand der vorhandenen Röntgenkristallstruktur identifiziert werden. Liegt keine Kristallstruktur vor, so kann mittels Computeranalysen versucht werden, anhand der vorliegenden Primärstruktur oberflächenexponierte Beta-Faltblattbereiche und die Zugänglichkeit einzelner Aminosäurepositionen vorherzusagen (www.embl-heidelberg.de/predictprotein/predictprotein.html) oder die 3d-Proteinstruktur zu modellieren (www.expasy.ch/swissmo/SWISS-MODEL.html) und damit Aussagen über möglicherweise oberflächenexponierte Aminosäuren zu gewinnen.

Es sind aber auch Mutagenesen im Beta-Faltblatt möglich, bei denen eine zeitaufwendige Vorauswahl der zu mutagenisierenden Aminosäurepositionen entfällt. Diejenigen DNA-Bereiche, die für die Beta-Faltblatt-Strukturen kodieren, werden aus ihrer DNA-Umgebung isoliert, einer zufallsgesteuerten Mutagenisierung unterzogen und anschließend wieder in die für das Protein kodierende DNA, aus der sie zuvor entnommen wurden, integriert. Hieran schließt sich ein Selektionsverfahren auf Mutanten mit den gewünschten Bindungseigenschaften und/oder katalytischen Eigenschaften und/oder Fluoreszenzeigenschaften an.

In einer weiteren Ausführungsform der Erfindung werden, wie oben bereits ausgeführt, die oberflächennahen Beta-Faltblatt-Regionen ausgewählt und innerhalb dieser ausgewählten Regionen die zu mutagenisierenden Aminosäurepositionen identifiziert. Diese so ausgewählten Aminosäurepositionen können dann auf DNA-Ebene entweder zielgerichtet mutagenisiert werden, d.h. ein für eine bestimmte Aminosäure kodierendes Codon wird durch ein Codon ausgetauscht, das für eine andere, vorher ausgewählte spezifische Aminosäure kodiert, oder dieser Austausch erfolgt im Rahmen einer Random-Mutagenese, wobei die auszutauschende Aminosäureposition festgelegt ist, nicht dagegen das für die neue, noch unbestimmte Aminosäure kodierende Codon.

Oberflächenexponierte Aminosäuren sind dem umgebenden Lösungsmittel zugänglich. Bei einer Zugänglichkeit der Aminosäuren im Protein von mehr als 8% im Vergleich zur Zugänglichkeit der Aminosäure im Modelltripeptid Gly-X-Gly sprechen wir von oberflächenexponierten Aminosäuren. Diese Proteinbereiche bzw. einzelne Aminosäurepositionen sind auch bevorzugte Bindungsstellen für mögliche Bindungspartner, auf welche erfindungsgemäß selektiert werden soll. Bei den Bindungspartnern kann es sich beispielsweise um Antigene oder Substrate oder Substrat-Übergangszustandsanaloga handeln.

Erfindungsgemäß können praktisch alle Proteine mutagenisiert werden, die an der Oberfläche liegende, einem Lösungsmittel oder einem Bindungspartner zugängliche Beta-Faltblatt-Strukturen zeigen. Hierbei kommen vorwiegend solche Proteine in Betracht, die besonders stabil sind, d.h. z. B. gegen Denaturierung resistent sind oder ausreichend "klein" sind.

Unter "Mutagenisierung" ist erfindungsgemäß die Veränderung einer oder mehrerer oberflächenexponierter Aminosäuren in der Polypeptidkette mit Beta-Faltblatt-Struktur zu verstehen. Hierunter fallen beispielsweise Aminosäuresubstitutionen, wobei eine Aminosäure mit bestimmten Eigenschaften in Bezug auf ihre Polarität, Ladung, Löslichkeit, Hydrophobizität oder Hydrophilizität durch eine Aminosäure mit einer anderen Eigenschaft ersetzt wird, beispielsweise also eine nicht polare hydrophobe Aminosäure durch eine polare Aminosäure, eine negativ geladene Aminosäure durch eine positiv geladene Aminosäure usw. Der Ausdruck "Mutagenisierung" umfaßt auch Insertionen und Deletionen einer oder mehrerer Aminosäuren. Voraussetzung dabei ist, daß die Mutationen oberflächenexponierte Aminosäuren in mindestens zwei oberflächenexponierten Beta-Strängen mindestens eines oberflächenexponierten Beta-Faltblatts umfassen. Die Mutationen werden bevorzugt gezielt an einzelnen Aminosäurepositionen im Beta-Faltblatt oder in ausgewählten Bereichen des Beta-Faltblatts gesetzt. Die Mutagenisierungen können in einem Bereich oder in mehreren Bereichen der Beta-Faltblatt-Struktur vorliegen. Die Veränderungen können benachbarte Aminosäuren oder weiter entfernt voneinander liegende Aminosäuren im Beta-Faltblatt umfassen. Die Veränderungen können auch Aminosäuren in verschiedenen Beta-Faltblättern, also in mehr als einem Beta-Faltblatt, umfassen. Die Insertionen, Deletionen oder Substitutionen von ein oder mehreren Aminosäuren sind in mindestens zwei oberflächenexponierten Beta-Strängen mindestens eines oberflächenexponierten Beta-Faltblatts lokalisiert. Dabei können in einem oberflächenexponierten Beta-Strang ein oder mehrere Aminosäuren substituiert, deletiert oder insertiert sein, d.h. ein oberflächenexponierter Beta-Strang kann mehrere Mutationen aufweisen, sofern mindestens zwei oberflächenexponierte Beta-Stränge mutiert sind. In einer weiteren Ausführungsform sind mindestens zwei oberflächenexponierte Beta-Faltblätter in jeweils einem oberflächenexponierten Beta-Strang mutagenisiert, d.h. jeweils ein oberflächenexponiertes Beta-Faltblatt weist zumindest einen mutagenisierten oberflächenexponierten Beta-Strang auf. In einer weiteren Ausführungsform der Erfindung sind die mutagenisierten oberflächenexponierten Beta-Faltblätter antiparallel angeordnet, wobei es sich bevorzugt um mindestens zwei antiparallel angeordnete Beta-Faltblätter handelt.

Es ist erfindungsgemäß beispielsweise bevorzugt, daß zwei oder drei oberflächenexponierte Beta-Stränge mutagenisiert sind. Es ist erfindungsgemäß auch möglich, daß vier oder mehr oberflächenexponierte Beta-Stränge mutgenisiert sind. Weiterhin ist es möglich, daß mindestens zwei Beta-Stränge in mindestens zwei Beta-Faltblättern mutagenisiert sind, wobei eine Mutagenese von drei Beta-Strängen in zwei antiparallelen Beta-Faltblättern bevorzugt ist.

In einer Ausführungsform der Erfindung erfolgt die Mutagenese durch Assemblierung von DNA-Oligonukleotiden mit dem Aminosäure-Codon NNK. Selbstverständlich sind auch andere Codons (Tripletts) einsetzbar.

Die Mutationen sind so, daß die Beta-Faltblatt-Struktur erhalten bleibt. Generell findet die Mutagenese an der Außenseite eines stabilen, an der Oberfläche des Proteines exponierten Beta-Faltblatt-Bereiches statt. Sie umfaßt sowohl site-spezifische als auch zufallsgesteuerte Mutagenisierungen. Ortsspezifische Mutagenesen, die einen relativ kleinen Bereich in der Primärstruktur umfassen (ca. 3-5 Aminosäuren), können mit den kommerziell angebotenen Kits von Stratagene (QuickChange) oder Bio-Rad (Muta-Gene phagemid in vitro mutagenesis kit) durchgeführt werden (vgl. US-A-5,789,166; US-A-4,873,192).

Bei größeren Bereichen einer ortsspezifischen Mutagenese muß eine DNA-Kassette hergestellt werden, wobei der zu mutagenisierende Bereich durch Assemblierung von Oligonukleotiden, die die mutierten und die nicht veränderten Positionen enthalten, erhalten wird (Nord et al., 1997; McConell und Hoess, 1995). Zufallsgesteuerte Mutagenesen können durch Vermehrung der DNA in Mutator-Stämmen oder durch eine PCR-Amplifikation (error-prone-PCR) eingeführt werden (z.B. Pannekoek et al., 1993). Dabei wird eine Polymerase mit erhöhter Fehlerrate verwendet. Um den Grad der eingeführten Mutagenese zu erhöhen, bzw. unterschiedliche Mutationen zu kombinieren, können die Mutationen in den PCR-Fragmenten mittels DNA shuffling (Stemmer, 1994) kombiniert werden. Eine Übersicht über diese Mutagenesestrategien bei Enzymen bietet das Review von Kuchner und Arnold (1997). Um diese zufallsgesteuerte Mutagenese in einem ausgewählten DNA-Bereich durchzuführen, muß ebenfalls eine DNA-Kassette konstruiert werden, die für die Mutagenese genutzt wird.

Die im Mutageneseschritt erhaltenen DNA-Moleküle werden in einem geeigneten Expressionssystem exprimiert. Bevorzugt sind solche Expressionssysteme, die die nachfolgende Selektion und Isolierung von Mutanten mit den gewünschten Bindungseigenschaften und/oder der gewünschten katalytischen bzw. enzymatischen Aktivität erleichtern. Derartige Expressionsvektoren und Expressionssysteme sind dem Fachmann bekannt und wurden bereits vorstehend näher beschrieben. Es ist selbstverständlich möglich, auch andere Expressionssysteme einzusetzen, die die erfindungsgemäße Selektion auf Mutanten mit spezifischen Eigenschaften bzw. Aktivitäten erlauben.

Bevorzugt wird zur Expression und Selektion das Phage-display-System eingesetzt, bei dem die Gesamtheit der auf DNA-Niveau erstellten Mutanten in ein Phagemid kloniert und auf der Oberfläche von Phagen exprimiert wird. Bei Proteinen mit reduzierten Cysteinen kann in einer besonders bevorzugten Ausgestaltungsform der Erfindung zur Verbesserung der Exposition und Selektion der Mutanten GSH zugegeben werden.

Die Erfindung erfaßt die mutagenisierten Proteine, DNA-Moleküle, hiervon abgeleitete RNA-Moleküle und funktionelle Teile hiervon, welche für ein Protein mit einer mutagenisierten Beta-Faltblatt-Struktur kodieren, das zu einer neuen oder veränderten Bindung an einen gewünschten Bindungspartner oder zu einer neuen oder veränderten katalytischen Aktivität mit einem Substrat befähigt ist oder neue oder veränderte Fluoreszenzeigenschaften aufweist. Der Ausdruck "funktionelle Teile" bezieht sich auf Untereinheiten, Domänen, und Epitope des Proteins mit Beta-Faltblatt-Struktur, die erfindungsgemäß mutagenisiert wurden und die die gewünschten Bindungseigenschaften und Aktivitäten besitzen bzw. hierfür mitverantwortlich sind.

Die Selektion und Isolierung von Mutanten mit den gewünschten Bindungseigenschaften und/oder den gewünschten katalytischen Aktivitäten und/oder Fluoreszenzeigenschaften erfolgt in an sich bekannter Weise. Beispiele für Selektionsverfahren und Isolierungsverfahren auf Mutanten mit neuen bzw. veränderten Bindungseigenschaften und neuen oder veränderten katalytischen Aktivitäten sind nachfolgend beschrieben:

Bei der Selektion auf gewünschte Bindungseigenschaften werden die mutierten Proteine bzw. die funktionellen Teile hiervon mit ihren Bindungspartnern in Kontakt gebracht. Durch geeignete Nachweisverfahren werden Mutanten mit den gewünschten Bindungseigenschaften selektiert.

Bei der Selektion auf katalytische Aktivität werden die mutierten Proteine oder funktionelle Teile hiervon mit den Substraten in Verbindung gebracht und anschließend durch geeignete Nachweismethoden auf die gewünschte enzymatische Aktivität selektiert.

Eine Selektion auf katalytische Aktivität kann in mehreren Ansätzen erfolgen:

### 1. Phage Display:

Kopplung von Übergangszustandsanaloga an eine Festphase und Selektion der Mutantenbank dagegen. Diese Substanzen sind Analoga zu Übergangszuständen des Substrates, die normalerweise bei einem enzymatischen Umsatz eines Substrates zum Produkt entstehen (Substrat-Übergangszustandsprodukt). Dafür muß der Übergangszustand des Substrates allerdings aufgeklärt sein. Es ist auch möglich, ein Screening auf Bindung des Substrates durchzuführen.

### 2. ohne Phage Display:

Klonierung der Mutanten in ein bakterielles Expressionssystem und Ausplattieren der rekombinanten Bakterien zum Bildung von Einzelkolonien. Das mutierte Protein kann in den Bakterien durch Zugabe von Induktoren (z.B. IPTG) zum Nährmedium exprimiert werden. Das Nährmedium muß weiterhin das Substrat enthalten, auf dessen Umsatz gescreent werden soll. Das Substrat muß bei Umsatz ein identifizierbares, z.B. farbiges Produkt bilden. Die Bakterien, die eine Mutante exprimieren, die das Substrat im Nährmedium umsetzt, erhalten dadurch eine andere Farbe. Ein Beispiel wäre das Screening auf Beta-Galactosidase-Aktivität und der Umsatz von X-Gal (Blaufärbung) (Zhang et al., 1997).

### 3. Dem Fachmann sind auch weitere Nachweisverfahren bekannt:

Neben der Variante der Farbbildung könnten z.B. Proteinmutanten selektiert werden, die eine neue Resistenz vermitteln (Zugabe von Antibiotika zum Nährmedium) oder die ein Wachstum auf minimalen Nährmedien ermöglichen, auf denen das "normale" Bakterium nicht wächst. Hier kann der selektive Wachstumsvorteil der Bakterien mit der neuen Proteinmutante ausgenutzt werden (Crameri et al., 1997).

### 4. Expression und Sekretion der mutierten Proteine:

z.B. in Bakterien, Gewinnung der Überstände und Prüfung auf die gewünschte, zu selektierende enzymatische Aktivität (You und Arnold, 1996). Die vorliegende Erfindung löst somit das Problem der Schaffung von Proteinen mit neuen Bindungseigenschaften oder neuen katalytischen Eigenschaften dadurch, daß Proteine mit Beta-Faltblatt-Strukturen in diesem Strukturmotiv mutagenisiert werden. Es wird auf solche Proteine selektiert, die die gewünschten neuen oder veränderten, vorzugsweise verbesserten Bindungseigenschaften oder die gewünschten neuen oder veränderten, vorzugsweise verbesserten, enzymatischen bzw. katalytischen Aktivitäten besitzen. Durch das erfindungsgemäße System ist es sogar möglich, Beta-Faltblatt-Proteine, die keine Bindungseigenschaften oder keine enzymatischen Eigenschaften besitzen, so zu verändern, daß sie nach Mutagenisierung im Beta-Faltblatt Bindungseigenschaften oder katalytische Eigenschaften erhalten.

Unter "Bindungseigenschaften" ist erfindungsgemäß beispielsweise die spezifische Affinität eines Antigens mit einem Antikörper zu verstehen. Nach der erfindungsgemäß durchgeführten Mutagenese besitzt das Beta-Faltblatt-Protein damit antikörperähnliche Eigenschaften und vereinigt in sich die Vorteile der hohen Bindungsspezifität eines Antikörpers mit den vorteilhaften Stabilitätseigenschaften eines Beta-Faltblatt-Proteins. Die erfindungsgemäß hergestellten Beta-Faltblatt-Proteine mit antikörperähnlichen Eigenschaften können auch eine katalytische Funktion besitzen.

Der erfindungsgemäße Lösungsvorschlag ermöglicht aber auch die Schaffung von Proteinen mit Beta-Faltblatt-Struktur mit neuen oder veränderten katalytischen Aktivitäten. Auch die Veränderung weiterer Proteineigenschaften, z.B. der fluoreszierenden Eigenschaften beim GFP, wäre möglich.

Unter Veränderung der Bindungseigenschaften, der katalytischen Aktivität oder der Fluoreszenzeigenschaften ist erfindungsgemäß sowohl eine Verschlechterung als auch eine Verbesserung dieser Eigenschaften zu verstehen, wobei eine Verbesserung bevorzugt ist.

Erfindungsgemäß wird unter "Protein mit einer neuen spezifischen Eigenschaft" oder "mit einer neuen katalytischen Aktivität" ein Protein verstanden, welches vorher keine spezifische Bindungseigenschaft oder keine katalytische Aktivität besaß und durch die gezielte Mutagenisierung oberflächenexponierter Aminosäuren in mindestens zwei oberflächenexponierten Beta-Strängen mindestens eines oberflächenexponierten Beta-Faltblatts nunmehr eine spezifische Bindungseigenschaft oder eine katalytische Aktivität oder eine Kombination aus beiden besitzt. Hierunter fallen aber auch Proteine, die bereits vor der Mutagenisierung eine spezifische Bindungseigenschaft oder eine katalytische Aktivität aufwiesen und nach der Mutagenisierung im Beta-Faltblatt eine weitere, zusätzliche spezifische Bindungseigenschaft und/oder katalytische Aktivität besitzen. Es ist selbstverständlich auch möglich, daß ein Protein mit einer spezifischen Bindungseigenschaft nunmehr eine katalytische Aktivität aufweist oder umgekehrt.

Von der Erfindung umfaßt werden weiterhin solche Proteine, die bereits eine spezifische Bindungseigenschaft und/oder eine enzymatische bzw. katalytische Aktivität und/oder Fluoreszenzeigenschaften besitzen und die nach der Mutagenisierung oberflächenexponierter Aminosäuren in mindestens zwei oberflächenexponierten Beta-Strängen eines oder mehrerer oberflächenexponierter Beta-Faltblätter eine Verbesserung, allgemeiner ausgedrückt, eine Veränderung ihrer spezifischen Bindungseigenschaften und/oder ihrer katalytischen Aktivität und/oder ihrer Fluoreszenzeigenschaften erhalten.

Das erfindungsgemäße Verfahren und die hierdurch hergestellten Proteine unterscheiden sich auch insofern von Proteinen und Verfahren aus dem Stand der Technik, bei denen die Beta-Faltblatt-Struktur durch Random-Mutagenisierungen verändert wurden, die nicht gezielt auf die Beta-Faltblatt-Struktur ausgerichtet waren, sondern auf das gesamte Protein und insbesondere nicht zielgerichtet auf oberflächenexponierte Aminosäuren in mindestens zwei oberflächenexponierten Beta-Strängen mindestens eines oberflächenexponierten Beta-Faltblatts gerichtet waren oder derartige oberflächenexponierte Aminosäuren betrafen.

In einer bevorzugten Ausführungsform der Erfindung, die nachfolgend beispielhaft beschrieben wird, wurde als ein Beispiel eines Proteins mit Beta-Faltblatt-Struktur das Gamma-Kristallin als Ausgangspunkt für die Mutagenese gewählt. Hierbei wurden zunächst durch Strukturuntersuchungen oberflächenexponierte Aminosäurepositionen ausgewählt und durch an sich bekannte Mutagenisierungsmethoden mutagenisiert. Die erhaltenen Mutanten wurden in einem geeigneten, ebenfalls bekannten Expressionssystem exprimiert. Die Selektion richtete sich auf solche Mutanten, deren oberflächenexponierte Aminosäuren im Beta-Faltblatt des Gamma-Kristallins eine spezifische Bindung an das Antigen BSA-Estradiol-17-Hemisuccinat zeigten. Es wurden zwar mehrere Mutanten mit der gewünschten Bindungseigenschaft isoliert, allerdings trägt nur eine die erwarteten Aminosäureaustausche. Es wurde damit ein antikörperähnliches, nicht Immunglobulin-Molekül erhalten, welches auf dem Ausgangsprotein Gamma-Kristallin basiert.

Durch das erfindungsgemäße Verfahren ist die Herstellung einer in der Tat riesigen Vielzahl von Mutanten möglich. Bereits die Mutagenese von acht Aminosäurepositionen ermöglicht die Bildung von 2,6 x 10¹⁰ verschiedenen Proteinspezies, die auf die gewünschten Bindungseigenschaften und katalytischen Aktivitäten hin untersucht werden können.

Es wurde erfindungsgemäß weiterhin gezeigt, daß ein Protein mit Beta-Faltblattstruktur durch Mutagenese von oberflächenexprimierten Aminosäuren in seinen Fluoreszenzeigenschaften verändert werden kann.

Die erhaltenen mutierten Gene können in geeigneten Systemen vermehrt und die Proteine exprimiert werden. Geeignete Expressionssysteme sind prokaryontische oder eukaryontische Systeme. Die für das mutierte Protein kodierende DNA wird beispielsweise in einen geeigneten Vektor, beispielsweise in einen Expressionsvektor, überführt und durch Transformation, Transfektion oder Infektion in eine Wirtszelle eingebracht. Die Verbindung mit regulatorischen Sequenzen, die die Expression der heterologen mutierten DNA gezielt steuern, ist selbstverständlich vorteilhaft.

Als Wirtszelle kann eine höhere Eukaryontenwirtszelle, beispielsweise eine Säugerzelle, oder eine niedere Eurkaryontenwirtszelle, beispielsweise eine Hefezelle, oder eine Prokaryontenzelle, beispielsweise eine Bakterienzelle, eingesetzt werden. Ein Beispiel für eine mögliche bakterielle Wirtszelle ist E.coli oder B.subtilis. Auch zellfreie Translationssysteme zur Herstellung der Proteine unter Verwendung von RNA, die sich von der DNA der vorliegenden Erfindung ableitet, ist möglich. Geeignete Klonierungs- und Expressionssysteme sind in verschiedenen Handbüchern zur Molekularbiologie, Biotechnologie und Gentechnologie beschrieben. Beispiele hierfür sind Sambrook et al., 1989 und Ausubel et al., 1994.

Die vorstehend allgemein dargestellte Erfindung wird nachfolgend anhand eines Ausführungsbeispiels und der beiliegenden Zeichnungen näher erläutert. Das Beispiel ist als eine mögliche Form der Erfindung zu verstehen, und die Erfindung ist nicht auf diese besondere Ausgestaltung beschränkt.

### Die beiliegenden Abbildungen zeigen:

- Abb. 1:: Oligonukleotide für die Assemblierung der Gamma-Kristallin-Mutanten.
- Abb.2:: Schematische Darstellung der Oligonukleotidassemblierung und nachfolgender PCR an Streptavidin beladenen Magnetic Beads (MB). Die mit X gekennzeichneten Stellen markieren die randomisierten Aminosäurepositionen.
- Abb. 3:: Schematische Darstellung der Amplifizierung der nicht mutagenisierten Region des Gamma-II-Kristallins.
- Abb. 4:: Oligonukleotide für die Amplifizierung der nicht mutagenisierten Region des Gamma-II-Kristallins.
- Abb. 5:: Schematische Darstellung der pCANTAB 5E- Gamma-II-Kristallin-Expressionskassette. g3-SS: Signalpeptidsequenz des Phagenproteins G3; E-Tag: 11 Aminosäuren für den immunologischen Nachweis; fd Gen 3: minor coat Protein 3 des filamentösen Phagen M13.
- Abb. 6:: Polyklonaler Phagen-ELISA mit angereicherten Phagen nach dem 3. Panning. Die Mikrotiterplatten wurden entweder mit dem Konjugat BSA-Beta-Estradiol 17-Hemisuccinat oder als Kontrolle nur mit BSA beschichtet. Nebeneinander dargestellt ist die Bindung der Gamma-II-Kristallin-Wildtyp-Phagen (GC-WT), der Phagen aus der Ausgangsbank (GCUC-1) und der durch wiederholtes Panning angereicherten Phagen (E-17 Phagen) an das jeweilige Antigen.
- Abb.7:: Partielle DNA-Sequenz der BSA-Estradiol-17-Hemisuccinat bindenden Gamma-II-Kristallin-Mutante 12A (Mu 12A) im Phagemid pGCKT 8-3 bzw. des Gamma-II-Kristallin Wildtyps (WT) in pCANTAB 5E. Kursiv und unterstrichen sind die eingeführten Schnittorte Sfi I (5') bzw. Bst Ell (3') markiert. Fett gedruckt sind die Kodons der randomisierten Aminosäurepositionen.
- Abb.8:: Abgeleitete Aminosäuresequenzen der BSA-Estradiol-17-Hemisuccinat bindenden Gamma-II-Kristallin-Mutante 12A (Mu 12A) und des Gamma-II-Kristallin Wildtyps (WT) nach Expression in den Phagemiden undAbspaltung des Signalpeptides. Die randomisierten Aminosäurepositionen sind fett und tatsächlich ausgetauschte Aminosäuren fett und unterstrichen gekennzeichnet. Durch den Sfi I-Schnittort N-terminal zusätzlich eingeführten Aminosäuren und die C-terminale E-Tag-Fusion sind kursiv und unterstrichen dargestellt.
- Abb. 9:: Sequenz der für die Klonierung von Mu 12A und Gamma-II-Kristallin in den Vektor pET-20b verwendeten Primer.
- Abb. 10:: Abgeleitete Proteinsequenz der BSA-Estradiol-17 bindenden Mutante 12A und des Gamma-II-Kristallins nach Expression in pET-20b. Die randomisierten Aminosäurepositionen sind fett und tatsächlich ausgetauschte Aminosäuren fett und unterstrichen gekennzeichnet. Durch die Klonierung zusätzlich eingeführte C-terminale Aminosäuren einschließlich der 6 Histidinreste sind kursiv und unterstrichen dargestellt.
- Abb. 11:: Konzentrationsabhängige Bindung der Mutante 12A an das Konjugat BSA-Beta-Estradiol 17-Hemisuccinat. Untersucht wurde die Bindung der Mutante (12A) und des Gamma-II-Kristallins (WT) an das Konjugat (BSA-Estr. 17) und als Kontrolle an BSA.

- Abb. 12:: Stabilität der Mutante 12A gegenüber dem Denaturierungsmittel Guanidin. Dargestellt sind die Emissionsmaxima nach Inkubation der gereinigten Proteine der Mutante 12A und des Gamma-II-Kristallins mit verschiedenen Konzentrationen an Guanidin und nach verschiedenen Zeiten.
- Abb. 13:: Fluoreszenzemissionsspektrum von Wildtyp Gamma-Kristallin und Mutante 12A in 50mM Na-Phosphat, pH 6,5. Das Fluoreszenzsignal (Abb.13A) wurde bei einer Anregungswellenlänge von 280 nm gemessen. Die Proteinkonzentration betrug 100 µg/ml. Abb. 13B zeigt die Absorptionsspektren der zur Fluorszenzmessung verwendeten Proteinproben. Die Absorption wurde in einer Küvette mit 1 cm Schichtdicke bestimmt.

### BEISPIEL

### Herstellung einer Gamma-Kristallin-Mutante mit spezifischer Bindung an das Hormon Estradiol

Das Design von neuartigen Beta-Faltblatt-Proteinen mit Antigenbindungseigenschaften wird anhand der Isolierung einer Mutante des *bovinen* Gamma-B-Kristallins (Gamma-II), die spezifisch an das Hormon Estradiol bindet, gezeigt. Durch gezielte Veränderung von ausgewählten Aminosäurepositionen eines oberflächenexponierten Beta-Faltblattes wurde ein neuartiges, stabiles Protein mit Beta-Faltblattstruktur und spezifischen Bindungseigenschaften hergestellt. Nach Auswahl der für eine Mutagenese geeigneten Region bzw. Aminosäuren im Beta-Faltblatt erfolgt eine ortsspezifische Mutagenese auf DNA-Ebene, die Herstellung einer Beta-Faltblatt-Mutanten-Bank in einem Phagemid, welches die Expression und nachfolgende Selektion auf neuartige Bindungseigenschaften der Mutanten im *Phage Display-*System ermöglicht. Die isolierte Mutante wurde hinsichtlich ihrer neuen Eigenschaften mit dem Ausgangsprotein Gamma-II-Kristallin verglichen.

### Auswahl einer geeigneten Region für die Mutagenese im Gamma-Kristallin

Basierend auf der Röntgenstruktur des Gamma-II-Kristallins (Wistow et al., 1983) wurde die N-terminale Domäne des Gamma-II-Kristallins (Acc. M16894) für eine Mutagenese ausgewählt. Insgesamt wurden dort acht Aminosäuren identifiziert, die ein zusammenhängendes Oberflächensegment ausbilden. Die ausgewählten Aminosäuren sind Bestandteil eines Beta-Faltblatts und sollten nicht maßgeblich zur Strukturerhaltung beitragen. Es handelt sich um Aminosäurepositionen, die dem Lösungsmittel und somit auch möglichen Bindungspartnern zugänglich sind. Die acht Aminosäuren Lys 2, Thr 4, Tyr 6, Cys 15, Glu 17, Ser 19, Arg 36 und Asp 38 umfassen eine Fläche von ca. 6,1% der Gesamtoberfläche des Proteins.

### Herstellung eines DNA-Pools an mutierten Gamma-II-Kristallin-Genen

Durch eine ortsspezifische Mutagenese wurden die acht Aminosäurepositionen randomisiert. Damit können 2,6 x *10¹⁰* verschiedene Proteinspezies gebildet werden. Der zu mutagenisierende Bereich wurde auf DNA-Ebene durch Zusammenlagerung einzelner Oligonukleotide erhalten. Nachfolgend erfolgte die Klonierung in ein für die Selektion im *Phage Display*-System konstruiertes Phagemid.

### Oligoassemblierung

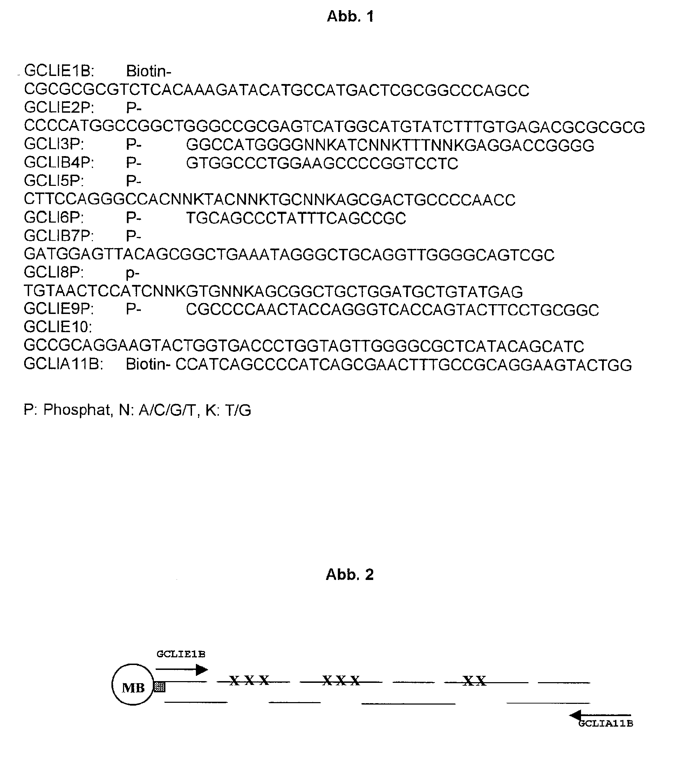

Für die Mutagenese wurden 227 bp, die den 5'-Bereich der Gamma-Kristallin-Mutanten mit den acht randomisierten Aminosäurepositionen sowie geeignete Restriktionsschnittorte enthielten, an einer Festphase assembliert. Insgesamt wurden dafür 10 einzelne Oligonukleotide verwendet, wovon drei die randomisierten Aminosäurepositionen enthielten (Abb. 1). An den 8 zu mutagenisierenden Positionen wurde bei der Primersynthese das Nukleotidgemisch NN(T/G) eingesetzt, wodurch theoretisch an einer Position 32 verschiedene Kodons entstehen (vgl. Nord et al., 1997). Zu Beginn der Assemblierung wurden biotinylierte Oligonukleotide an mit Streptavidin beladene Magnetic Beads (MBs) der Firma Dynal (M-280) angelagert. Nach mehreren Anlagerungs-, Ligations- und Polymeraseschritten konnte der an der Festphase assemblierte Pool an mutagenisierten Regionen des Gamma-Kristallins durch PCR amplifiziert werden (Abb. 2). Die ca. 250 bp langen PCR-Produkte enthielten 5' einen Sfi 1- und 3' einen Bst Ell-Schnittort.
Alle für die Assemblierung verwendeten Oligonukleotide wurden auf eine Konzentration von 100 pmol/µl eingestellt. Zuerst wurden die Primer GCLIE1B und GCLIE2P assembliert. Dafür wurden jeweils 4 µl der Primer mit 36 µl Wasch- und Bindungspuffer (WB-Puffer: 1M NaCl, 10 mM Tris-HCl pH 7,5, 1 mM EDTA) versetzt und für 5 min bei 70°C inkubiert. Nach Vereinigung der beiden Primer und weiterer 5-minütiger Inkubation bei 70°C wurde das Primergemisch langsam auf Raumtemperatur abgekühlt. 4 µl des GCLIE1B/GCLIE2P-Primerhybrids wurden mit 56 µl WB-Puffer vermischt und zu 300 µg der Streptavidinbeladenen MBs, die zuvor mit Wasch- und Bindungspuffer gewaschen wurden, gegeben. Nach einer 15-minütigen Inkubation bei Raumtemperatur erfolgte ein Waschen der MBs mit WB- und TE-Puffer (10 mM Tris-HCl pH 7,5, 1 mM EDTA). Zu den mit dem 1. Primerhybrid gekoppelten MBs wird ein Primer-Brückenfragment gegeben, welches wie folgt hergestellt wird: 4 µl des Primers GCLIB4P bzw. GCLI5P werden mit 36 µl 1 x Ligationspuffer der Fa. GIBCO BRL (50 mM Tris-HCl pH 7,6, 10 mM MgCl₂, 1 mM ATP, 1 mM DTT, 5% (w/v) Polyethylenglycol-8000) vermischt. Nach einer 5-minütigen Inkubation bei 70°C werden beide Ansätze vereinigt, weitere 5 min bei 70°C inkubiert und auf Raumtemperatur abgekühlt. Zu dem Gemisch werden 4 µl GCLI3P und 16 µl 1 x Ligationspuffer, die zuvor wiederum für 5 min bei 70°C inkubiert wurden, gegeben. Nach Zugabe von 12 units T4-DNA-Ligase (Fa. GIBCO BRL) und 8 µl 1 x Ligationspuffer wird der Reaktionsansatz für 1 h bei Raumtemperatur inkubiert. 12 µl dieses GCLIE3P/GCLIB4P/GCLI5P-Brückenfragmentes werden mit 54 µl 1 x Ligationspuffer sowie 6 units Ligase versetzt, zu den gewaschenen MBs, die das erste Primerhybrid enthalten, gegeben und für 1 h bei Raumtemperatur inkubiert. Nach der Ligationsreaktion werden die MBs zweimal mit TE-Puffer gewaschen und in 64 µl 1 x Ligationspuffer mit 8 µl Ligase aufgenommen. Die MBs werden dann mit 8 µl des assemblierten Primergemisches GCLI6P/ GCLIB7P versetzt, deren Assemblierung zuvor analog der von GCLIB4P/GCLI5P erfolgte. Die Ligation wurde wiederum für 1 h bei Raumtemperatur durchgeführt. Nach einem zweimaligen Waschen der MBs in TE-Puffer werden 12 µl des 2. Brückenfragmentes GCLIB8P/GCLIE9P/GCLIE10 zugegeben und für 1 h ligiert. Die Herstellung des 2. Brückenfragmentes erfolgt analog zum 1. Brückenfragment, wobei GCLIE9P und GCLIE10 zuerst assembliert und im zweiten Schritt mit GCLI8P ligiert werden.
Die MBs mit den immobilisierten Primern werden danach wiederum mit TE-Puffer gewaschen. Die nachfolgende DNA-Polymerase- und Ligasereaktion füllt die Lücken im Zweitstrang auf. Die MBs werden dafür für 30 min bei 37°C in dem folgenden Puffergemisch inkubiert: 52,5 µl H₂O 6 µl Puffer L Fa. Boehringer (100 mM Tris-HCl pH 7,5, 100 mM MgCl₂, 10 mM Dithioerythritol), 0,5 µl dNTP's (25 mM jedes dNTP) und 1 µl 2 units Klenow-Fragment (Fa. Boehringer). Die Ligationsreaktion schließt sich nach zweimaligen Waschen der MBs mit TE-Puffer für 1 h bei Raumtemperatur an. In einem 100 µl-Ansatz befinden sich 10 units Ligase. Nach einem zweimaligen Waschschritt mit TE-Puffer wird der nicht kovalent an die MBs gebundene DNA-Strang durch Behandlung mit 40 µl 0,1 M NaOH für 30 s entfernt und die MBs in 60 µl TE resuspendiert. Die PCR zur Amplifizierung der Bank wird mit den MBs als Template durchgeführt. Die PCR-Reaktion (50 µl) wird wie folgt angesetzt: 6 µl MBs, 5 µl 10 x PCR-Reaktionspuffer der Fa. Stratagene (100 mM KCl, 100 mM (NH₄)₂SO₄, 200 mM Tris-HCl pH 8,75, 20 mM MgSO₄, 1% Triton X-100, 1 mg/ml BSA), 1 µl (2,5 units) Pfu-DNA Polymerase (Fa. Stratagene), 0,5 µl dNTP's (25 mM jedes dNTP), 0,35 µl GCLIE1B, 0,35 µl GCLIA11B und 36,8 µl H₂O. Die PCR erfolgte in 35 Zyklen mit einem Primer-Annealing bei 55°C für 1 min, einer Polymerasereaktion bei 72°C für 1,5 min, einer Denaturierung bei 95°C für 1 min und einer abschließenden Polymerase-Reaktion für 5 min bei 72°C.

### Herstellung des Phagemids pGCKT 8-3

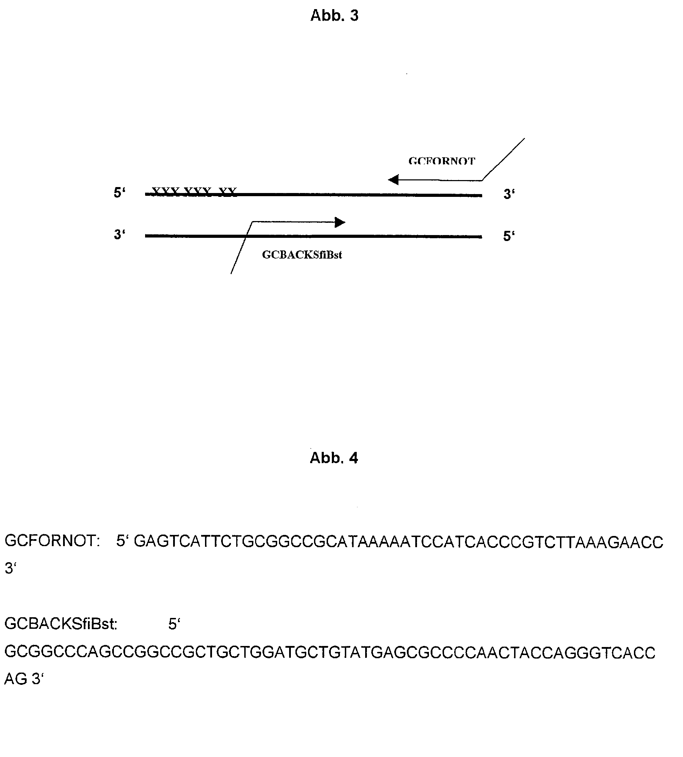

Ausgehend von dem Phagemid pCANTAB 5E (PRAS-Kit der Fa. Pharmacia Biotech) wurde ein Phagemid-Derivat für die Klonierung der Gamma-II-Kristallin-Mutanten-Bank konstruiert. Der gesamte 3'-Bereich des Gamma-II-Kristallins (C-terminale Domäne) und der nichtmutagenisierte 5'-Bereich wurden mittels PCR unter Verwendung des Plasmids pGII (Mayr et al., 1994) als Template und der Primer GCFORNOT und GCBACKSfiBst amplifiziert (Abb. 3, 4).

Die durch die Primer eingeführten Sfi I- (GCBACKSfiBst) bzw. Not I-Schnittorte (GCFORNOT) ermöglichen die Insertion des PCR-Produktes in das Phagemid pCANTAB 5E. Mit dem Primer GCBACKSfiBst wurde zusätzlich ein Bst Ell-Schnittort in das Gamma-Kristallin-Gen integriert, der die Klonierung der mutierten Gamma-Kristallin-DNA-Fragmente erlaubt. Die de novo-Einführung des Schnittortes führt nicht zu einer Veränderung der Aminosäuresequenz im Gamma-II-Kristallin. Das PCR-Produkt wurde nach Sequenzierung als Sfi I/Not I-Fragment in das Sfi I/Not I geschnittene Phagemid pCANTAB 5E kloniert. Das so konstruierte Phagemid pGCKT8-3 war Ausgangspunkt für die Herstellung der Gamma-II-Kristallin *Phage Display*-Bank.

### Herstellung der Gamma-Kristallin-Mutanten-Bank und Klonierung des Wildtyp-Gamma-II-Kristallins

Das Phagemid pGCKT 8-3 wurde mit den Restriktionsenzymen Bst Ell und Sfi I geschnitten und einer Phosphatasebehandlung unterzogen (*shrimps* Phosphatase, Fa. USB). Die DNA wurde nach den einzelnen Spaltungen gelelektrophoretisch aufgetrennt, die gespaltenen Vektor-Fraktionen ausgeschnitten und mittels Elektroelution aus den Agarosegelen isoliert. Vor jeder weiteren enzymatischen Behandlung erfolgte eine Phenol/Chloroform-Extraktion und eine Fällung der DNA mit Glycogen. Der mittels PCR amplifizierte DNA-Fragmentpool, der die mutierte Region des Gamma-II-Kristallins enthielt, wurde mit den Restriktionsenzymen Sfi I und Bst Ell gespalten. Für die Ligation der PCR-Produkte in das vorbereitete Phagemid pGCKT 8-3 wurden insgesamt 440 ng Phagemid und 110 ng PCR-Produkt eingesetzt. Die Ligationen erfolgten mit insgesamt 44 units T4-DNA-Ligase (Fa. GIBCO BRL) in 20 µl-Ansätzen bei 16°C über Nacht. Nach einer 20 minütigen Inaktivierung der Ligase bei 70°C wurden die Ligationsreaktionen für 1 h durch Tropfendialyse entsalzt. Jeweils 30 µl elektrokompetenter *E. coli*-TG 1-Zellen wurden mit je 15 µl der dialysierten Ligationen transformiert. Die Herstellung der elektrokompetenten Zellen und die Transformation erfolgten wie im Manual des PRAS-Kit beschrieben. Die transformierten Zellen wurden auf den Glukose- und Ampicillin- (100 µg/ml) enthaltenden SOBAG-Platten (siehe Manual des PRAS-Kits, Fa. Pharmacia-Biotech) ausplattiert und bei 30°C über Nacht inkubiert. Die hergestellte Bank GCUC-1 umfaßte 250.000 Originalklone. Die Klone wurden mit 2 x YT-Medium (siehe Manual der PRAS-Kits), welches 1 % Glucose und 20% Glycerin enthielt, abgeschwemmt, aliquotiert und bei -80°C gelagert. Der Amplifikationsfaktor der Bank wurde mit 7x10⁶ bestimmt. Der Anteil an rekombinanten Klonen in der GCUC-1-Bank betrug 97%. Die Sequenzierung zufällig ausgewählter Klone ergab, daß an den randomisierten Aminosäurepositionen Kodons mit der erwarteten Varianz eingesetzt wurden. Mittels Western-Blot-Analysen wurden in der Bank Expressionsraten von 30-60% nachgewiesen.

Für Kontrollexperimente wurde die Gamma-II-Kristallin-DNA wurde mit den Primern GCFORNOT (5' GAGTCATTCTGCGGCCGCATAAAAATCCATCACCCGTCTTAAAGAACC 3') und GCBACKSFI (5' CATGCCATGACTCGCGGCCCAGCCGGCCATGGGGAAGAT CACTTTTTACGAGGAC 3') und dem Plasmid pGII (Mayr et al., 1994) als *template* amplifiziert. Das sequenzierte PCR-Produkt wurde nach Spaltung mit den Restriktionsendonukleasen Not I und Sfi I in das ebenfalls Sfi I/Not I geschnittene Phagemid pCANTAB 5E kloniert.

### Aufbau des Phage Displays und Selektion auf neuartige Bindungseigenschaften

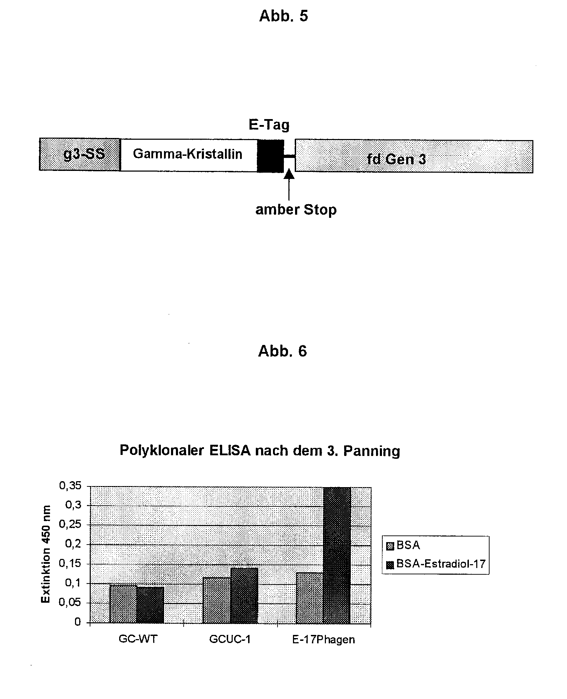

Zur Selektion von Gamma-Kristallin-Mutanten auf Bindungseigenschaften wurde das kommerziell erhältliche *Phage Display*-System PRAS der Fa. Pharmacia-Biotech verwendet.
In dem verwendeten Phagemiden pCANTAB 5E (Wildtyp-Gamma-II-Kristallin) und pGCKT 8-3 (Gamma-Kristallin-Mutanten) sind die Gamma-Kristalline N-terminal mit dem G3-Signalpeptid und C-terminal mit einem E-Tag, der den immunologischen Nachweis der Proteine ermöglicht, fusioniert (Abb. 5). In Abhängigkeit vom verwendeten Bakterienstamm wird das dem E-Tag folgende *amber* Stop-Kodon entweder erkannt (*E. coli*-HB 2151) und die Gamma-Kristalline werden nach Abspaltung des Signalpeptides ins Periplasma sekretiert oder überlesen (*E. coli TG* 1) und die Gamma-Kristalline werden als Fusion mit dem *minor* coat Protein des filamentösen Phagen M 13 synthetisiert und nach Abspaltung des Signalpeptides in der inneren Plasmamembran der *E*. *coli*-Zelle verankert. Nach Zugabe eines Helferphagen kömen rekombinante Phagen gebildet werden, die auf ihrer Oberfläche die Gamma-II-Kristallin-Varianten exponieren.

### Optimierung der Anzuchtbedingungen für die GCUC-1-Bank und die Gamma-II-Kristallin-Wildtyp-Phagen

Unter den im PRAS-Manual beschriebenen Anzuchtbedingungen wurden keine rekombinanten Phagen erhalten, bei denen mittels Western-Blot-Analysen die erwarteten Fusionsproteine (Gamma-II-Kristallin/Protein 3) nachgewiesen werden konnten. Erst durch Zugabe von reduziertem Glutathion (GSH) während der Phagenbildung wurde der Redoxzustand im Periplasma der Bakterienzelle verändert und somit günstigere Bedingungen für die Phagenassemblierung geschaffen. Bei Verwendung des Gamma-II-Kristallin-Klons konnten rekombinante, das Fusionsprotein tragende Phagen nur bei Zugabe von GSH nachgewiesen werden. Mit steigender GSH-Konzentration stieg auch der Anteil der Gamma-II-Kristallin-Phagen. Die optimale GSH-Konzentration wurde mit 8 mM bestimmt. Eine Ursache für die schlechte Expression des Gamma-Kristallins auf der Phagenoberfläche ohne Zugabe von GSH könnte der hohe Anteil an reduzierten Cysteinen (7) im Gamma-Kristallin sein. Bei Eintritt des teilweise entfalteten Gamma-Kristallins in das Periplasma könnte es unter den dort vorherrschenden oxidativen Bedingungen zur Misfaltung und Aggregatbildung durch Disulfidbrückenbildung kommen. Dadurch könnte auch die Phagenassemblierung unterdrückt werden. Bei Verwendung von Proteinen mit reduzierten Cysteinen im *Phage Display*-System kann möglicherweise generell durch Zugabe von GSH die Bildung rekombinanter Phagen verbessert werden.

### Selektionsprozeß mit der GCUC1-Phagen Display-Bank

Für das Screening der GCUC-1-Bank wurde alle verwendeten Glasgeräte für 4 h bei 200 °C und Plastik-Material mit Helipur für 1 h sterilisiert. Das Panning der GCUC-1-Bank erfolgte mit BSA-Beta-Estradiol 17-Hemisuccinat (Fa. Sigma) als Antigen. Für das Panning wurden Mikrotiterplatten (Maxisorp Fa. NUNC) als Festphase verwendet. Die Stringenz der Waschschritte wurde im Verlauf der 3 Panningrunden gesteigert. Für die erste Anzucht wurden 100 ml 2 x YT-Medium mit 2% Glukose und Ampicillin (100 µg/ml) mit 50 µl der GCUC-1 Bank inokuliert. Die Bakterien wuchsen bei 37°C und 300 rpm bis zu einer OD₆₀₀ von 0,4. In 10 ml dieser Bakterienkultur wurden 800 µl M13K07-Helferphage (1 x 10¹¹pfu/ml, GIBCO BRL) gegeben. Es folgte eine Inkubation bei 37°C für 30 min ohne und für weitere 30 min mit leichtem Schütteln (50 rpm). Das Bakterienpellet wurde durch Zentrifugation für 20 min, 1.500 rpm (Sorvall SS 34 Rotor) bei Raumtemperatur erhalten und in 100 ml 2 x YT-Medium mit 8 mM GSH, 100 µg/ml Ampicillin und 50 µg/ml Kanamycin aufgenommen. Die Bildung der rekombinanten Phagen erfolgte durch Übernacht-Kultivierung bei 30 °C und 300 rpm. Der Überstand mit den rekombinanten Phagen wurde durch eine zweimalige 15-minütige Zentrifugation bei 10.800 g und eine anschließende Filtration (Porengröße 0,45 µm) erhalten. Die Konzentrierung der Phagen erfolgte durch Zugabe von 1/5 PEG/NaCl-Lösung (20% PEG-8000, 2,5 M NaCl) zum Überstand, einer einstündigen Inkubation auf Eis, sowie zweimaliger 30 minütiger Zentrifugation bei 4°C und 3.300 g. Das erhaltene Phagenpellet wurde in 4 ml PBS pH 7,2 suspendiert und restliche Zellbestandteile durch eine Zentrifugation (10 min, 11.600 g, Raumtemperatur) abgetrennt.Für den Selektionsprozeß (Panning) wurde 1 ml der konzentrierten Phagen mit 1 ml einer 6%igen BSA-Lösung (6 % BSA in PBS, pH 7,2) vermischt und für 10 min bei Raumtemperatur inkubiert. Jeweils 100 µl der so behandelten Phagen wurden zu den wie folgt vorbereiteten, Antigen-beschichteten Mikrotiterplattenvertiefungen, gegeben. NUNC-Maxisorp-Mikrotiterplatten wurden über Nacht mit dem Antigen BSA-Beta-Estradiol 17-Hemisuccinat beschichtet. Insgesamt wurden 10 Vertiefungen mit je 100 µl der Antigenlösurg (100 µg/ml in PBS pH 7,6) versetzt. Die über Nacht bei Raumtemperatur beschichteten Vertiefungen wurden dreimal mit PBS, pH 7,6 gewaschen. Das Absättigen freier Bindungsstellen erfolgte durch Füllung der Vertiefungen mit einer 3%igen BSA/PBS, pH 7,2-Lösung für 2 h bei Raumtemperatur. Vor Zugabe der BSAbehandelten Phagen wurden die Vertiefungen zweimal mit einer PBS-Lösung (pH 7,2) gewaschen. Das Panning erfolgte durch 30-minütiges leichtes Bewegen der Mikrotiterplatte (20 rpm) und eine nachfolgende 90-minütige Inkubation ohne Schütteln bei Raumtemperatur. Unspezifisch gebundene Phagen wurden durch 10-maliges Waschen mit PBS, pH 7,2/0,1% Tween-20 und 10 maliges Waschen mit PBS, pH 7,2 entfernt. Bindende Phagen wurden durch Zugabe und 10-minütige Inkubation bei Raumtemperatur von jeweils 100 µl 100 mM Triethylamin (frisch hergestellt) pro Vertiefung eluiert. Eine Neutralisierung der basisch eluierten Phagen (1 ml) erfolgte durch Zugabe von 500 µl 1 M Tris-HCl pH 7,4. 750 µl dieser Phagen wurden für die Infektion von 9 ml, auf Minimalmedium-Platten angezogenen TG-1-Zellen mit einer OD₆₀₀ von 0,4-0,5 verwendet. Dafür wurden die Bakterien mit den Phagen für 30 min bei 37°C inkubiert. Besonders starke Binder, die durch die Triethylamin-Behandlung nicht von der Mikrotiterplatte entfernt wurden, konnten durch direkte Infektion von TG-1-Zellen gesichert werden. Dafür wurden jeweils 100 µl der angezogenen TG-1-Zellen zu den Vertiefungen gegeben. Nach einer 30-minütigen Inkubation bei 37°C wurden die infizierten TG-1-Zellen abgenommen und mit denen der Infektion mit den eluierten Phagen vereinigt. Die infizierten Bakterien wurden auf 16x16 cm-SOBAG-Platten ausplattiert und über Nacht bei 30°C inkubiert. Jeweils 1 µl der konzentrierten und eluierten Phagen wurde für die Titer-Bestimmung verwendet. Die erhaltenen Bakterienklone wurden mit 12,5 ml 2 x YT, 20% Glycerin von den SOBAG-Platten abgeschwemmt. Das 2. und 3. Panning erfolgte analog zum ersten mit den folgenden Änderungen. Die erneute Phagenanzucht erfolgte mit 20 µl der abgeschwemmten Bank in 20 ml Medium. 2 ml der angezogenen Bakterienkultur wurden für die Infektion mit dem Helferphagen verwendet (Verhältnis Bakterien/Phagen: 1/20). Das Waschen der Mikrotiterplatten erfolgte beim 2. Panning zuerst 15 mal mit PBS/Tween-20 und dann 10-mal mit PBS und beim 3. Panning zuerst 20-mal mit PBS/Tween-20 und dann 10-mal mit PBS.

### ELISA zur Überprüfung der Anreicherung und spezifischen Bindung

Der Nachweis der Anreicherung spezifisch bindender Phagen an das Antigen erfolgte mit einem polyklonalen Phagen-ELISA. Neben den eluierten Phagen wurden Phagen der Ausgangsbank GCUC-1 und des Wildtyp-Gamma-II-Kristallins als Vergleich getestet. NUNC-Maxisorp-Platten wurden mit 100 µl BSA-Estradiol-17-Hemisuccinat oder BSA in einer Konzentration von 2 µg/ml PBS pH 7,6 über Nacht bei Raumtemperatur beschichtet. Nach 3 maligem Waschen der Vertiefungen mit PBS, pH 7,6 erfolgte ein Blocking für 2 h bei 37°C mit 3% Trockenmilchpulver (Glücksklee)/PBS, pH 7,2 und nochmaliges (3 x) Waschen mit PBS, pH 7,6. Die nach der Phagenanzucht isolierten, nicht konzentrierten, rekombinanten Phagen wurden zunächst für 1 h bei Raumtemperatur geblockt (1:1 Mischen mit 6%igem Trockenmilchpulver (Marvel) / PBS pH 7,6. 100 µl der geblockten Phagen wurden pro Vertiefung aufgetragen und für 1 h bei 37°C inkubiert. Nach einem jeweils 3-maligen Waschen der Vertiefungen mit PBS/Tween-20 und PBS erfolgte eine Inkubation mit dem anti-M 13-Antikörper-POD-Konjugat (Fa. Pharmacia-Biotech, Verdünnung 1:5000 in 3% Glücksklee/PBS) für 1 h bei 37°C. Nach Waschen der Platten erfolgte der Nachweis des enzymgebundenen Antikörpers mit 100 µl Immuno-Pure-TMB-Substrat (Fa. Pierce). Die Farbreaktion wurde durch Zugabe von 100 µl 2M H₂SO₄ abgestoppt und die Extinktion bei 450 nm bestimmt. Das Ergebnis der Anreicherung von Phagen nach dem 3. Panning, die an das BSA-Estradiol-Konjugat binden, ist in Abb. 6 dargestellt.

### Isolierung und Charakterisierung von Einzelphagen mit spezifischer Bindung an das Konjugat

Von den nach dem 3. Panning erhaltenen Bakterienklonen wurden 80 Einzelklone ausgewählt. Von den Klonen wurden Phagen isoliert und im monoklonalen Phagen-ELISA hinsichtlich ihrer Antigenbindung einzeln getestet. Die Anzucht von einzelnen Bakterienklonen erfolgte in 100 µl 2 x YT-Medium mit 2% Glukose und 100 µg/ml Ampicillin über Nacht bei leichtem Schütteln (100 rpm) in Polypropylen-Mikrotiterplatten (Fa. NUNC). 2 µl dieser Bakterienkulturen wurden 1:100 in dem gleichen Medium verdünnt und bei 100 rpm bis zu einer OD₆₀₀von 0,4 bei 37°C kultiviert. Phagen wurden, wie für den Selektionsprozeß beschrieben, erhalten. Für die Phagenanzucht wurdendeep *well*-Polypropylen-Mikrotiterplatten der Fa. TECAN verwendet. Für den ELISA wurden 200 µl des nach Zentrifugation erhaltenen Phagenüberstandes (nicht konzentriert) mit 40 µl 6xPBS/18% Marvel für 1 h bei Raumtemperatur geblockt. Von den 80 getesteten Klonen zeigten 30 eine deutliche Bindung der rekombinanten Phagen an BSA-Estradiol-17 und nicht an das parallel getestete BSA. Phagen mit dem Wildtyp-Gamma-II-Kristallin zeigte in einem Kontrollexperiment keinerlei Bindung an BSA-Estradiol-17. 14 ausgewählte Binder wurden mit den IRD 800 markierten Primern pCANR1LAB (5' CCATGATTACGCC-AAGCTTTGGAGCC 3') und GCLISEQ (5' CTGAAAGTGCCGGTGTGTTGC 3') sequenziert. Die Sequenzierung ergab nur in einem Fall eine Gamma-Kristallin-Variante (Mu 12A), die ausschließlich in den acht randomisierten Aminosäurepositionen mutiert war. Eine Reihe von Klonen zeigte Verschiebungen im Leserahmen, die zwar theoretisch für ein funktionelles Protein, allerdings nicht ausschließlich Veränderungen in der erwarteten Region des Gamma-Kristallins aufwiesen. Diese *frame shift*-Mutanten wurden nicht weiter verfolgt.

### Charakterisierung der Beta-Faltblatt-Mutante 12A

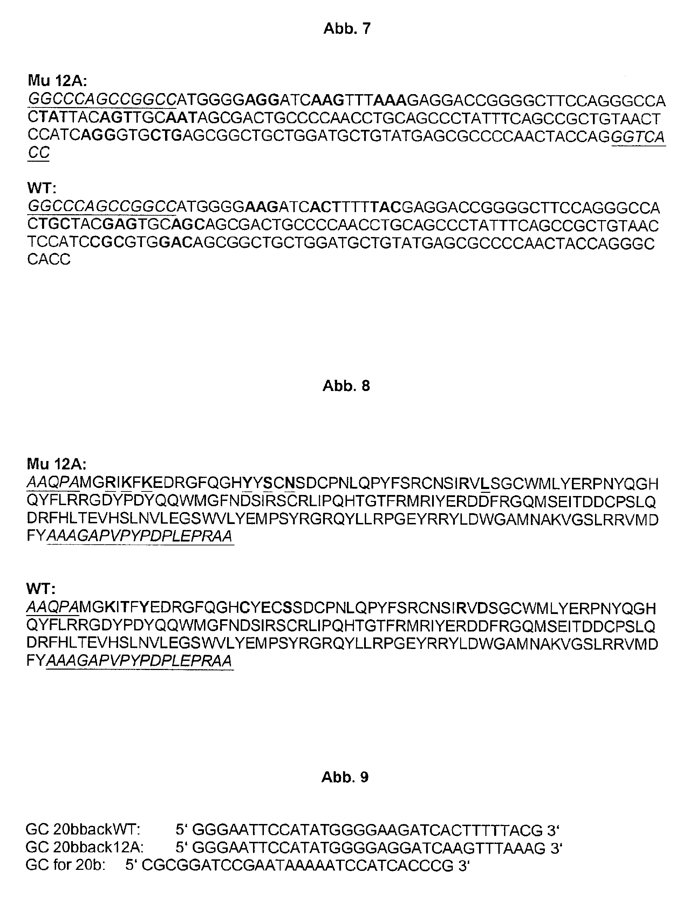

Die Expression des Fusionsproteins Mu 12A*-minor coat* Protein 3 auf der Oberfläche der rekombinanten Phagen sowie die Expression von Mu 12A in dem *E. coli*-Stamm HB 2151 wurde mittels Western-Blot-Analysen unter Verwendung des Anti-G3P- bzw. des Anti-E-Tag-Antikörpers (Fa. Pharmacia-Biotech) nachgewiesen. Die DNA-Sequenz der Mutante 12A im Phagemid pGCKT 8-3 und des Gamma-II-Kristallin-Wildtyps ist in Abb. 7 dargestellt. Die DNA-Sequenz beginnt mit dem Sfi I-Schnittort, der bereits im Ausgangsphagemid pCANTAB 5E vorhanden ist, und endet im Falle von pGCKT 8-3 mit dem in das Gamma-II-Kristallingen neu eingeführten Bst Ell-Ort bzw. der ursprünglichen Sequenz im Falle des Gamma-II-Kristallin-Wildtypgens. In Abb. 8 sind die daraus abgeleiteten Aminosäuresequenzen dargestellt. Die Kodon-Randomisierung in Aminosäureposition 36 führt nicht zu einer Veränderung der Aminosäure Arginin in dieser Position. Die Computer-Modellierung der Mutante 12A zeigt, daß die Aminosäureaustausche nicht zu großen Veränderungen in der Proteinstruktur im Vergleich zum Ausgangsprotein führen. Allerdings kommt es zu einer positiven Verschiebung der Nettoladung:

### Expression von Mu 12A in pET-20b

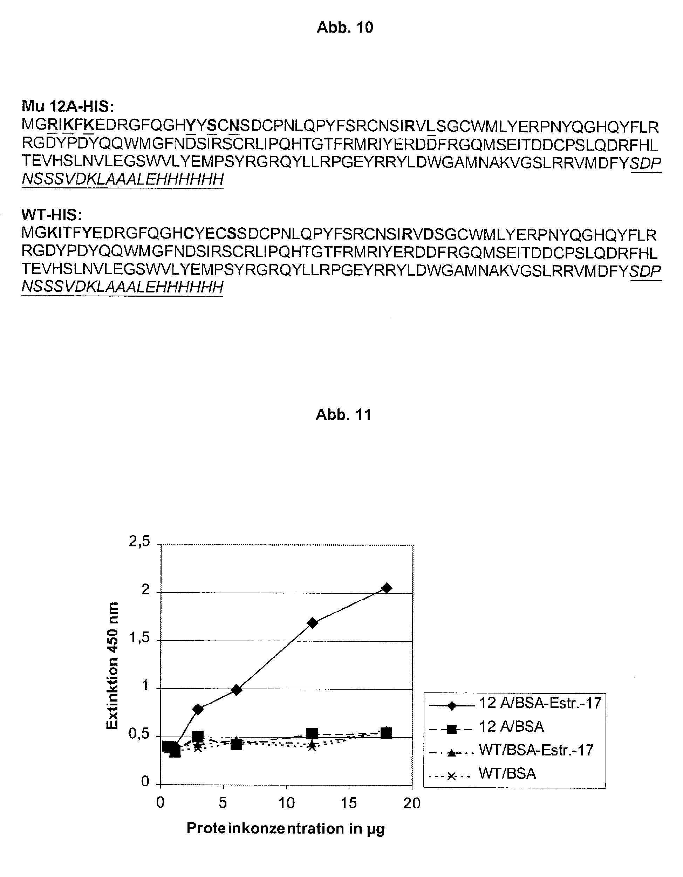

Für eine ausführliche Charakterisierung der Mutante 12A erfolgte eine Umklonierung der DNA in das Plasmid pET-20b (Fa. Novagen). Das Plasmid ermöglicht eine hohe Expression der rekombinanten DNA in dem *E*. *coli*-Stamm BL 21 sowie eine leichte Reinigung der Fremdproteine. Gene werden hier ohne Signalpeptid mit einer C-terminalen Fusion von 6 Histidinresten exprimiert. Die DNA der Mutante 12A und des Gamma-II-Kristallin-Wildtyps wurden mittels PCR unter Verwendung der entsprechenden Phagemid-DNA und der Primer GC 20bback12A/GC for 20b für die Mutante 12A und GC 20bbackWT/GC for 20b für den Wildtyp amplifiziert (Abb. 9). Die PCR-Fragmente wurden mit den Restriktionsendonukleasen Nde I und Bam Hl gespalten und in den Nde I/ Bam HI geschnittenen Vektor pET 20b kloniert. Die theoretische Aminosäuresequenz der Mutante 12 A bzw. des Gamma-II-Kristallins nach Expression in pET-20b in Abb. 10 dargestellt. Die ersten 10 N-terminalen Aminosäuren der Mutante 12 A wurde durch N-terminale Proteinsequenzierung bestätigt.

### Anzucht und Reinigung der Mutante und des Wildtyps in pET-20b

Für genaue Untersuchungen der Bindungseigenschaften und der Stabilität der Mutante wurden große Mengen an Protein der Mutante 12A und des Wildtyps hergestellt. BL 21-Zellen wurden mit den Plasmiden pET-20b/Mu 12A bzw. pET-20b/Gamma-II-Kristallin transformiert. Für die Anzucht der Klone wurde eine Vorkultur 1:100 mit LB-Medium/100 µg/ml Ampicillin verdünnt und die Kultur bei 37°C bis zu einer OD₆₀₀ von 0,5 bei 200 rpm geschüttelt. Die Expression der Gamma-Kristalline wurde durch Zugabe von IPTG (1 mM Endkonzentration) induziert. Die weitere Kultivierung erfolgte über Nacht bei 30°C und 200 rpm. Die Bakterienzellen wurden durch Zentrifugation bei 4°C, 6.000 rpm (Sorvall GS3 Rotor) für 10 min geerntet. Das Zellpellet wurde in 30 ml 2 x PBS unter Zugabe von 150 µl 200 mM PMSF und 10 µl DNAse (Fa. Boehringer) suspendiert. Es erfolgte ein zweimaliger Zellaufschluss mit der Gaulinpresse bei 800-1000 PSIG. Der Überstand mit den löslichen Proteinen wurde nach Zentrifugation der Zellsuspension für 1 h bei 4°C und 20.000 rpm (Sorvall SS 34 Rotor) erhalten. Die Reinigung der mit 6 Histidinresten fusionierten Gamma-Kristalline erfolgte über Affinitätschromatographie bei 4°C. 8 ml Ni-NTA wurden mit 50 ml 2 x PBS/10 mM Imidazol äquilibriert. Der Überstand mit den löslichen Proteinen wurde dann im Batch-Verfahren mit dem äquilibrierten Säulenmaterial über Nacht auf einem Rollenschüttler langsam bewegt. Nach Umfüllen der Suspension in eine Chromatographiesäule erfolgte ein Waschen mit 2 x PBS/10 mM Imidazol/300 mM NaCl. Die Elution des gebundenen Proteins erfolgte mit 2 x PBS/ 250 mM Imidazol. Dem eluierten Proteinen wurde DTT (Endkonzentration 10 mM) zugesetzt. Anschließend erfolgten 2 Dialyseschritte bei 4°C für jeweils 8 h: 1. mit 100 mM Na-Phosphatpuffer pH 6,0/ 1 mM EDTA/1 mM DTT und 2. mit 10 mM Na-Phosphatpuffer pH 6,0/ 1 mM EDTA. Der nach einer abschließenden Zentrifugation (4°C, 30 min, 20.000 rpm mit Sorvall SS 34 Rotor) erhaltene Überstand enthielt das gereinigte Protein (Mu 12A bzw. Gamma-II-Kristallin), das für Bindungs- und Stabilitätsuntersuchungen eingesetzt wurde.

Für die Testung der spezifischen Bindung der Mutante 12A an das Konjugat BSA-Estradiol-17-Hemisuccinat wurde ein ELISA durchgeführt, wobei steigende Konzentrationen an gereinigtem Protein der Mutante 12A-His-Tag eingesetzt wurden. Als Kontrolle wurden steigende Mengen an Gamma-II-Kristallin-Wildtyp (ebenfalls mit His-Tag) eingesetzt sowie die Bindung beider gereinigter Proteine an BSA getestet. Der konzentrationsabhängige ELISA wurde mit NUNC-Tm-Platten durchgeführt. Die Antigen-Beschichtung mit dem BSA-Estradiol-17-Hemisuccinat-Konjugat bzw. dem BSA erfolgte über Nacht bei Raumtemperatur. Die Beschichtung erfolgte mit jeweils 100 µl Antigen in einer Konzentration von 20 µg/ml PBS pH 7,6. Nach Waschen (2 x PBS pH 7,6) und Blocken der Platten (3% Marvel/PBS für 2 h bei 37°C) wurden jeweils 1-13 µl der Proteinstammlösung (Konzentration 0,63 mg/ml) an gereinigter Mu 12A bzw. Gamma-II-Kristallin in insgesamt 100 µl Reaktionslösung (PBS, 3% Marvel, x µl Protein) gegeben und für 2 h bei 37°C in den Vertiefungen inkubiert. Als sekundäre Antikörper wurden der Tetra His Antikörper der Fa. Qiagen in einer Verdünnung von 1:3000 und der Anti-Maus-POD-Antikörper (Fa. Sigma) in einer Verdünnung von 1:2000 eingesetzt. Die Antikörper wurden mit einer 3%igen Marvel/PBS-Lösung verdünnt, 100 µl zu den Vertiefungen gegeben und für jeweils 1 h bei 37°C inkubiert. Die Substratreaktion erfolgte wie für den polyklonalen Phagen-ELISA beschrieben. Das Ergebnis dieses ELISA in Abb. 11 zeigt deutlich, daß nur mit steigenden Konzentrationen der Mutante 12A steigende Extinktionen gemessen werden. Keinerlei Anstieg wurde mit dem Gamma-II-Kristallin festgestellt. Ebenfalls wurde keine Reaktion mit BSA beobachtet. Damit ist die spezifische Bindung der Mutante 12A im Vergleich zum Ausgangsprotein gezeigt.

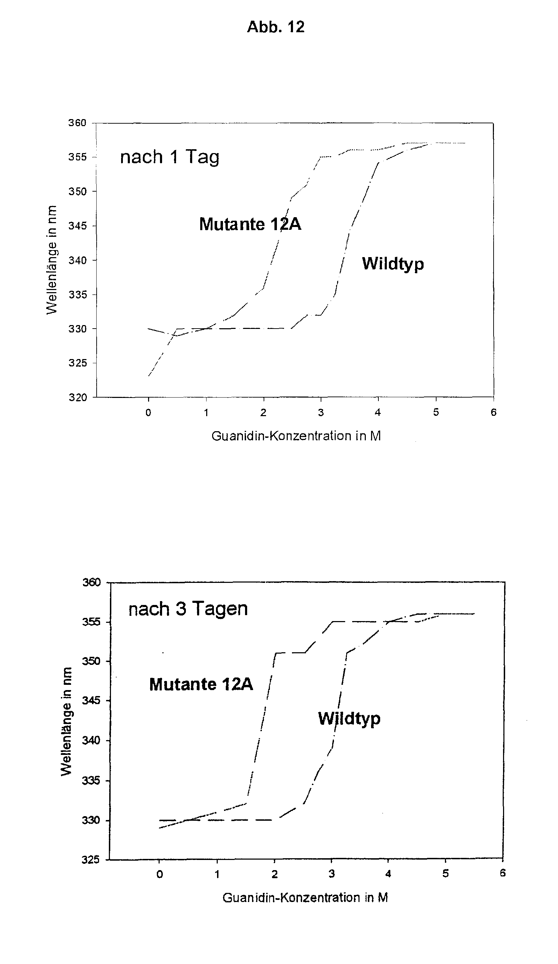

Für die Untersuchungen zur Stabilität wurden Guanidin-Denaturierungskurven der Mutante 12 A sowie des Gamma-II-Kristallins aufgenommen. Dazu wurden die gereinigten Proteine in einer Endkonzentration von 20 µg/ml mit steigenden Konzentrationen an Guanidinium für einen und für drei Tage bei 20°C inkubiert. Insgesamt wurden 15 Guanidiniumkonzen trationen im Bereich von 0 - 5,5 M in einer 1 mM DTT/0,1 M Na-Phosphat-Puffer pH 6,0-Lösung eingestellt. Von jedem Ansatz wurde nach einem bzw. drei Tagen ein Fluoreszenz-Emisssions-Spektrum von 300-400 nm aufgenommen. Die Anregungswellenlänge betrug 280 nm. In Abb. 12 sind die festgestellten Emissionsmaxima gegen die Guanidiniumkonzentrationen abgetragen. Die Stabilität des Gamma-II-Kristallins ist sowohl bei einem als auch bei drei Tagen höher als die der Mutante 12A. Im Vergleich zu Antikörpermolekülen weist die Mutante 12A jedoch eine viel höhere Stabilität auf.

### Veränderung der Fluoreszenzeigenschaften von Mutante 12A

Um zu prüfen, ob die Mutante 12A gegenüber dem Wildtypprotein veränderte Fluoreszenzeigenschaften besitzt, wurden Fluoreszenzspektren aufgenommen. Dazu wurden jeweils 100 µg/ml vom Wildtypprotein bzw. der Mutante 12A (in 50 mM Na-Phosphat, pH 6,0) bei 280 nm angeregt und die Fluoreszenz in einem Wellenlängenbereich von 300 und 400 nm in einer Küvette mit 1 cm Schichtdicke vermessen. Die Spaltbreite betrug sowohl für die Anregung als auch für die Emission 5 nm.

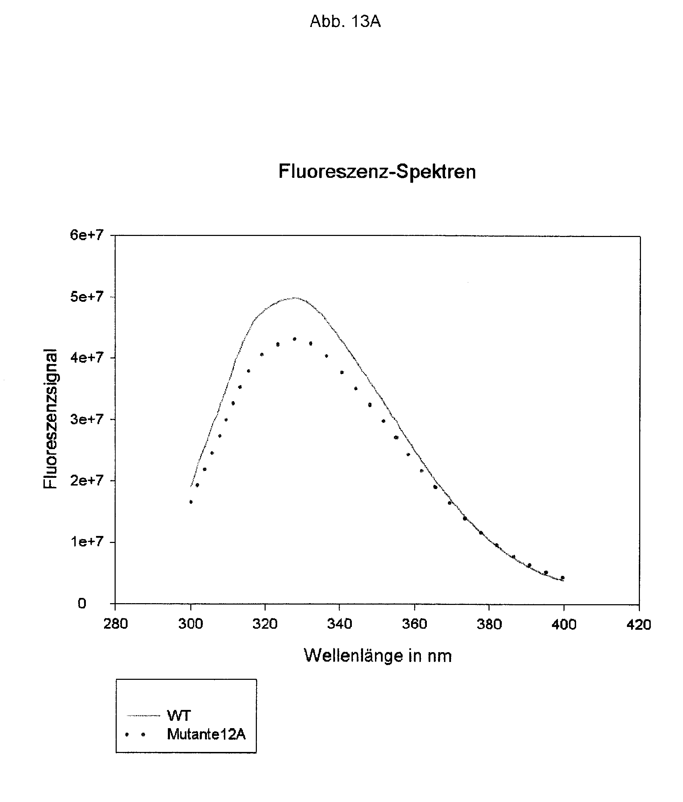

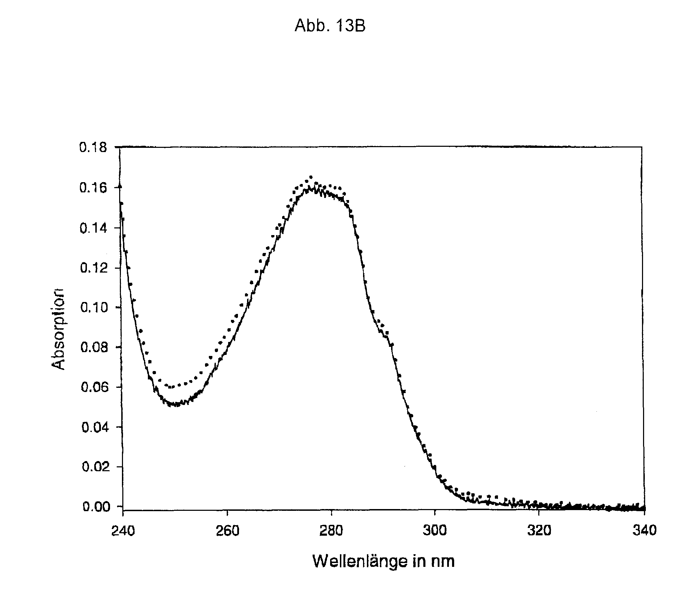

Das detektierte Fluorszenzsignal wies sowohl für den Wildtyp als auch für die Mutante 12A ein Maximum bei 329 nm auf. Jedoch war die Fluoreszenzintensität bei der Mutante 12A, mit nur 86% Signalintensität, deutlich geringer verglichen mit dem Gamma-Kristallin Wildtyp (100%) (s. Abb 13A).
Die Gesamtanzahl der Fluorophore sind in Mutante 12A und Wildtyp identisch. Jedoch bewirken die Sequenzveränderungen in der Mutante (Y -> K an Position 8 und C -> Y an Position 15) eine Änderung des Fluoreszenzsignals. Die Differenz in der Fluoreszenzintensität kann darauf zurückgeführt werden, daß der Tyrosinrest in Position 8 bzw. 15 verschiedene Fluoreszenzeigenschaften aufweist.

### Zitierte Literatur:

Bird, R. E., Hardman, K. D., Jacobson, J. W., Johnson, S., Kaufman, B. M., Lee, S.-M., Lee, T., Pope, H. S., Riordan, G. S. and Whitlow, M. (1988): Single-chain antigen-binding proteins. Science 242, 423-426.

Brinkmann, U., di Carlo A., Vasmatzis, G., Kurochkina, N., Beers, R., Byungkook, L. and Pastan, I. (1997): Stabilization of a recombinant Fv fragment by base-loop interconnection and VH-VL permutation. J. Mol. Biol. 268, 107-117.

Choo, Y. and Klug, A. (1995): Designing DNA-binding proteins on the surface of filamentous phage. Curr. Opin. Biotechnol. 6, 431-436.

Colcher, D., Pavlinkova, G., Beresford, G., Booth, B. J., Choudhury, A. and Batra, S. K. (1998): Pharmacokinetics and biodistribution of genetically-engineered antibodies. Q. J. Nucl. Med. 42, 225-241.

Cortese R., Monaci, P., Luzzago, A., Santini, C., Bartoli, F., Cortese, I., Fortugno, P., Galfre, G., Nicosia, A. and Felici, F. (1995): Selection of biologically active peptides by phage display of random peptide libraries. Curr. Opin. Biotechnol. 6, 73-80.

Cumber, J. A., Ward, E. S., Winter, G., Parnell, G. D. and Wawrzynczak, E. J. (1992): Comparative stabilities in vitro and in vivo of a recombinant mouse antibody FvCys fragment and a bisFvCys conjugate. J. of Immunology 149, 120-126.

Glockshuber, R., Malia, M., Pfitzinger I. and Plückthun, A. (1990): A comparison of strategies to stabilize immunoglobulin Fv-fragments. Biochemistry 29, 1362-1367.

Haaparanta T. and Huse W. D. (1995): A combinatorial method for constructing libraries of long peptides displayed by filamentous phage. Mol. Diversity 1, 39-52.

Hanes, J. et al. (1997): In vitro selection and evolution of functional proteins by using ribosome display. Proc. Natl. Acad. Sci. USA. 94, 4937-42.

Hazes, B. and Hol, W. G. J. (1992): Comparison of the hemocyaninß-barrel with other greek key β-barrels: possible importance of the "β-zipper" in protein structure and folding. Proteins: Struct., Funct. Gen. 12, 278-298.

Hemmingsen, J. M., Gernert, K. M., Richardson, J. S. and Richardson, D. C. (1994): The tyrosine corner: a feature of most greek key β-barrel proteins. Prot. Science 3, 1927-1937.

Holliger, H. and Winter G. (1993): Engineering bispecific antibodies. Curr. Opin. Biotech. 4, 446-449.

de Kruif, J., Boel, E. and Logtenberg, T. (1995): Selection and application of human single chain Fv antibody fragments from a semi-synthetic phage antibody display library with designed CDR3 regions. J. Mol. Biol. 248, 97-105.

Ku, J. and Schultz, P. G. (1995): Alternate protein frameworks for molecular recognition. Proc. Natl. Acad. Sci. USA 92, 6552-6556.

Mayr, E.-M., Jaenicke, R. and Glockshuber, R. (1994): Domain interactions and connecting peptides in lens crystallins. J. Mol. Biol. 235, 84-88.

Mandal, K., Chakrabart, B., Thomson, J. and Siezen, R. J. (1987): Structure and stability of β-crystallins. Denaturation and proteolysis behaviour. J. Biol. Chem. 262, 8096-8102.

McConell S. and Hoess R. H. (1995): Tendamistat as a scaffold for conformationally constrained phage peptide libraries. J. Mol. Biol. 250, 460-470.

McConell, S. J., Uveges, A. J., Fowlkes, D. M. and Spinella, D. G (1996): Construction and screening of M 13 phage libraries displaying long random peptides. Mol. Diversity 1, 165-176.

Nissim, A., Hoogenboom, H. R., Tomlinson, I. M., Flynn, G., Midgley, C., Lane, D. and Winter, G. (1994): Antibody fragments from a 'single pot' phage display library as immunochemical reagents. EMBO J. 13, 692-698.

Nord K., Gunneriusson, E., Ringdahl, J., Stahl, S., Uhlen, M. and Nygren, P. A. (1997): Binding proteins selected from combinatorial libraries of an β-helical bacterial receptor domain. Nat. Biotechnol. 8, 772-777.

Pack, P. and Plückthun, A. (1992): Miniantibodies: use of amphipathic helices to produce fuctional, flexibly linked dimeric Fv fragments with high avidity in Escherichia coli. Biochemistry 31, 1579-1584.

Pantoliano, M. W., Bird, R. E., Johnson, S., Asel, E. D., Dodd, S. W., Wood, J. F. and Hardman, K. D. (1991): Conformational stability, folding, and ligand-binding affinity of single-chain Fv immunoglobulin fragments expressed in Escherichia coli. Biochemistry 30, 10117-10125.

Richardson, J. S., Richardson, D. C., Tweedy, N. B., Gernert, K. M., Quinn, T. P., Hecht, M. H., Erickson, B. W., Yan, Y., McClain, R. D., Donlan, M. E. and Surles, M. C. (1992): Looking at proteins: representations, folding, packing and design. Biophys. J. 63, 1186-1209.

Riddle, D. S., Santiago, J. V., Bray-Hall, S. T., Doshi, N., Grantcharova, Q. Y and Baker, D. (1997): Functional rapidly folding proteins from simplified amino acid sequences. Nature structural biology 4, 805-809.

Rudolph, R., Siebendritt, R., Nesslauer, G., Sharma, A. and Jaenicke, R. (1990): Folding of an all-β protein: independent domain folding in GammaBll-crystallin from calf eye lens. Proc. Natl. Acad. Sci. USA 87, 4625-4629.

Slingsby, C.(1985): Structural variation in lens crystallins. TIBS 10, 281-284.

Smith, G. P (1985): Filamentous Fusion Phage: Novel expression vectors that display cloned antigens on the virion surface. Science 228, 1315-1317.

Stahl, S. and Uhlen, M. (1997): Bacterial surface display: trends and progress. TIBTECH 15, 185-192.

Wistow, G. J. and Piatigorsky, J. (1988): Lens crystallins: the evolution and expression of proteins for a highly specialized tissue. Ann. Rev. Biochem. 57, 479-504.

Wistow, G. J., Turnell, B., Summers, L., Slingsby, C. Moss, D., Miller, L., Lindley, P. and Blundell, T. (1983): X-ray analysis of the eye lens protein y-II-crystallin at 1.9 A° resolution. J. Mol. Biol. 170, 175-202.

Young, N. M., MacKenzie, C. R., Narang, S. A., Oomen, R. P. and Baenziger, J. E. (1995): Thermal stabilization of a single-chain Fv antibody fragment by introduction of a disulphide bond. FEBS Lett. 377, 135-139.

Ausubel, F.M., Brent, R., Kinston, R.E., Moore, D.D., Seidmann, J.G., Smith, J.A. and Struhl, K. (1994): Current protocols in molecular biology. John Wiley & Sons, Inc.

Crameri, A., Dawes, G., Rodriguez, E., Jr., Silver, S. and Stemmer, W.P. (1997): Molecular evolution of an arsenate detoxification pathway by DNA shuffling. Nat. Biotechnol. 5, 436-438.

Kuchner, O. and Arnold, F.H. (1997): Directed evolution of enzyme catalysts. TIBTECH 15, 523-530.

Pannekoek, H., van Meijer M., Schleef, R.R., Loskutoff, d.J. and Barbas, C.F. (1993): Functional display of human plasminogen-activator inhibitor 1 (PAI-1) on phages: Novel perspectives for structure-function analysis by error-prone DNA synthesis. Gene 128, 135-140.

Sambrook, J., Maniatis, T. and Fritsch, E.F. (1989): Molecular Cloning: A laboratory manual. Cold spring Harbor. Cold Spring Harbour Laboratory Press, New York.

Stemmer, W.P.C. (1994): Rapid evolution of a protein in vitro by DNA shuffling. Nature 370, 389-391.

You, L. and Arnold, F.H. (1996): Directed evolution of subtilisin E in Bacillus subtilis to enhance total activity in aqueous dimethylformamide. Protein Eng. 1, 77-83.

Zhang, J.H., Dawas, G. and Stemmer, W-P. (1997): Directed evolution of a fucosidase from a galctosidase by DNA shuffling and screening. Proc. Natl. Acad. Sci. U.S.A. 94, 4504-4509.

### Ausgestaltungen der Erfindung

1. Protein mit Beta-Faltblatt-Struktur,
   dadurch gekennzeichet, dass
   oberflächenexponierte Aminosäuren in mindestens zwei oberflächenexponierten β-Strängen mindestens eines oberflächenexponierten Beta-Faltblatts gezielt derart mutagenisiert sind, dass das Protein neue oder veränderte spezifische Bindungseigenschaften oder eine neue oder veränderte katalytische Aktivität oder neue oder veränderte Fluoreszenzeigenschaften aufweist.
2. Protein nach Ausgestaltung 1,
   dadurch gekennzeichnet, dass
   es zur Gruppe der Kristalline, Spheruline, Hitzestreßproteine, Kälteschockproteine, β-Helix-Proteine, Lipocaline, Certine, Fibronectine oder Transkriptionsfaktoren gehört oder GFP, NGF, Tendamistat oder Lysozym ist.
3. Protein nach Ausgestaltung 1 oder 2,
   dadurch gekennzeichnet,
   dass drei oberflächenexponierte Beta-Stränge mutagenisiert sind.
4. Protein nach Ausgestaltung 1 oder 2,
   dadurch gekennzeichnet,
   dass vier oder mehr oberflächenexponierte Beta-Stränge mutagenisiert sind.
5. Protein nach einer oder mehrerer der vorhergehenden Ausgestaltungen,
   dadurch gekennzeichnet,
   dass mindestens zwei Beta-Stränge in mindestens zwei Beta-Faltblättern mutagenisiert sind.
6. Protein nach einer oder mehrerer der vorhergehenden Ausgestaltungen,
   dadurch gekennzeichnet,
   dass drei Beta-Stränge in zwei antiparallelen Beta-Faltblättern mutagenisiert sind.
7. Protein nach einer oder mehrerer der vorhergehenden Ausgestaltungen,
   dadurch gekennzeichnet,
   dass es ein Kristallin aus Wirbeltieren, Nagern, Vögeln oder Fischen ist.
8. Protein nach einer oder mehrerer der vorhergehenden Ausgestaltungen,
   dadurch gekennzeichnet,
   dass es ein Alpha-, Beta- oder Gamma-Kristallin ist.
9. Protein nach einer oder mehrerer der vorhergehenden Ausgestaltungen,
   dadurch gekennzeichnet,
   dass es ein Gamma-II-Kristallin-Protein ist.
10. Protein nach einer oder mehrerer der vorhergehenden Ausgestaltungen,
   dadurch gekennzeichnet,
   dass das Protein in einem Lösungsmittel und/oder einem Bindungspartner zugänglichen Bereich des Beta-Faltblatts mutagenisiert ist.
11. Protein nach einer oder mehrerer der vorhergehenden Ausgestaltungen,
   dadurch gekennzeichnet,
   dass es in einer β-Faltblattstruktur einer Domäne oder einer Untereinheit des Proteins mutagenisiert ist.
12. Protein nach einer oder mehrerer der vorhergehenden Ausgestaltungen,
   dadurch gekennzeichnet,
   dass es ein Gamma-II-Kristallin ist, das durch Mutagenese einer oder mehrerer der Aminosäuren Lys 2, Thr 4, Tyr 6, Cys 15, Glu 17, Ser 19, Arg 36 und Asp 38 im Gamma-II-Kristallin erhalten wurde.
13. Protein nach einer oder mehrerer der vorhergehenden Ausgestaltungen,
   dadurch gekennzeichnet,
   dass das Protein im Beta-Faltblatt so mutagenisiert wurde, dass es antikörperähnliche Bindungseigenschaften oder eine enzymatische (katalytische) Aktivität aufweist.
14. Protein nach Ausgestaltung 12 oder 13,
   dadurch gekennzeichnet,
   dass es Bindungsspezifität für Estradiol oder dessen Konjugat BSA-β-Estradiol-17-Hemisuccinat aufweist.
15. Protein nach einer oder mehrerer der vorhergehenden Ausgestaltungen,
   dadurch gekennzeichnet,
   dass es Bindungsspezifität für Estradiol oder dessen Konjugat BSA-β-Estradiol-17-Hemisuccinat aufweist mit der nachfolgenden Aminosäurensequenz: (Vgl. Abbildungen 8 und 10.
16. Protein nach einer oder mehrerer der vorhergehenden Ausgestaltungen,
   dadurch gekennzeichnet,
   dass es mit anderen Proteinen oder Nicht-Protein-Substanzen kombiniert ist.
17. Protein nach einer oder mehrerer der vorhergehenden Ausgestaltungen,
   dadurch gekennzeichnet,
   dass es eine verbesserte Bindungseigenschaft und/oder verbesserte katalytische Aktivität und/oder verbesserte Fluroeszenzeigenschaft aufweist.
18. DNA, die für ein Protein nach einer oder mehrerer der vorhergehenden Ausgestaltungen kodiert.
19. RNA, die von der DNA nach Ausgestaltung 18 abgeleitet ist.
20. Prokaryotische oder eukaryotische Vektoren oder Zellen, enthaltend eine DNA oder RNA nach Ausgestaltung 18 oder 19 oder Teile hiervon, die für funktionelle Bereiche des Proteins kodieren.
21. Verfahren zur Herstellung eines Proteins nach einer oder mehrerer der vorhergehenden Ausgestaltungen mit den nachfolgenden Schritten:
   a. Mutagenese der für ein Protein mit Beta-Faltblatt-Struktur kodierenden DNA, in solchen Bereichen, die für mindestens zwei oberflächenexponierte Beta-Stränge eines oberflächenexponierten Beta-Faltblatts kodieren.
   b. Expression der in Schritt (a) erhaltenen Mutanten in einem geeigneten Expressionssystem; und
   c. Selektion und Isolierung von Mutanten mit den gewünschten Bindungseigenschaften und/oder der gewünschten katalytischen Aktivität; wahlweise
   d. Expression sowie Aufreinigung der im Beta-Faltblatt mutierten Proteine.
22. Verfahren nach Ausgestaltung 21,
   dadurch gekennzeichnet,
   dass die Mutagenese eine Mutagenese spezifischer Aminosäurepositionen (Ortspezifische Mutagenese) oder nichtspezifischer Aminosäurepositionen (Randommutagenese) im Beta-Faltblatt umfaßt.
23. Verfahren nach einer oder mehrerer der vorhergehenden Ausgestaltungen,
   dadurch gekennzeichnet,
   dass die Mutanten im Schritt b) in pro- oder eukaryontischen Zellen, im zellfreien System als Komplex mit Ribosomen oder auf der Oberfläche von pflanzlichen oder tierischen Zellen, Hefezellen oder Phagen, Viren oder Bakterien exprimiert werden.
24. Verfahren nach einer oder mehrerer der vorhergehenden Ausgestaltungen,
   dadurch gekennzeichnet,
   dass zur Selektion von Mutanten mit den gewünschten Bindungseigenschaften diese mit dem Bindungspartner in Kontakt gebracht und solche Mutanten isoliert werden, die die gewünschte Bindungsaffinität aufweisen.
25. Verfahren nach einer oder mehrerer der vorhergehenden Ausgestaltungen,
   dadurch gekennzeichnet,
   dass zur Selektion von Mutanten mit den gewünschten katalytischen Eigenschaften diese mit ihrem Substrat in Kontakt gebracht und solche Mutanten isoliert werden, die die gewünschte katalytische Aktivität aufweisen.
26. Verwendung eines Proteins nach einer oder mehrerer der vorhergehenden Ausgestaltungen in der Diagnostik und Therapie, in der Kosmetik, der Bioseparation und Biosensorik und der Schadstoffverringerung.

### Annex zu den Anmeldungsunterlagen - nachgereichtes Sequenzprotokoll

## Patentansprüche

1. Protein mit β-Faltblatt-Struktur mit antikörperähnlicher Bindungseigenschaft gegenüber einem Bindungspartner und/oder mit einer katalytischen Aktivität gegenüber einem Substrat,
wobei das Protein, dessen Kristallstruktur bekannt ist, ausgewählt ist aus der Gruppe der Kristalline, Spheruline, Hitzestreßproteine, Kälteschockproteine, Fibronektine und Beta-Helix-Proteine, und wobei im Protein durch Substitution, Deletion und/oder Insertion oberflächenexponierte Aminosäuren in zwei, drei oder vier oberflächenexponierten β-Strängen eines oberflächenexponierten β-Faltblatts mutagenisiert vorliegen, so dass das Protein an einen Bindungspartner bindet oder gegenüber einem Substrat eine katalytische Aktivität aufweist,
wobei die Bindung an das mutagenisierte Protein und die katalytische Aktivität gegenüber einem Substrat durch folgende Verfahrensschritte bestimmt wird:
a) Auswählen eines Proteins aus der vorstehend definierten Gruppe;
b) Auswählen eines Bindungspartners oder Substrats;
c) Bestimmen der oberflächenexponierten Aminosäuren des Proteins aus Schritt a)
d) Mutagenisieren der für das Protein mit β-Faltblatt-Struktur codierenden DNA durch Substitution, Deletion und/oder Insertion der oberflächenexponierten Aminosäuren in 2, 3 oder 4 oberflächenexponierten β-Strängen eines oberflächenexponierten β-Faltblatts;
e) Exprimieren der im Schritt d) erhaltenen Mutanten in einem geeigneten Expressionssystem;
f) in Kontakt bringen der im Schritt e) erhaltenen Proteine mit einem Bindungspartner oder Substrat aus Schritt b);
g) Selektieren und Isolieren von mutierten Proteinen, die mit dem im Schritt b) ausgewählten Bindungspartner oder Substrat unter Ausbildung einer neuen, oder verbesserten antikörperähnlichen Bindungseigenschaft oder katalytischen Aktivität binden,
wobei unter neuer, antikörperähnlicher Bindungseigenschaft gegenüber einem Bindungspartner und/oder neuer katalytischer Aktivität zu verstehen ist, dass das Protein aus Schritt a) ohne die Substitution, Deletion, oder Insertion keine Bindungseigenschaft gegenüber dem Bindungspartner und/oder keine katalytische Aktivität gegenüber dem Substrat aufweist und nach der Substitution, Deletion oder Insertion eine neue Bindungseigenschaft gegenüber dem Bindungspartner und/oder eine katalytische Aktivität gegenüber dem Substrat aufweist, oder
das Protein aus Schritt a) vor der Substitution, Deletion oder Insertion eine Bindungseigenschaft und/oder katalytische Aktivität aufweist und nach der Substitution, Deletion oder Insertion eine weitere neue, oder verbesserte antikörperähnliche Bindungseigenschaft gegenüber dem Bindungspartner und/oder eine weitere neue katalytische Aktivität gegenüber dem Substrat besitzt.

2. Protein nach Anspruch 1,
**dadurch gekennzeichnet,**
**dass** oberflächenexponierte Aminosäuren in mindestens zwei oder drei oberflächenexponierten Beta-Strängen mutagenisiert sind, oder dass vier oder mehr oberflächenexponierte Beta-Stränge mutagenisiert sind, oder dass in 1 oberflächenexponierten Beta-Strang ein oder mehrere Aminosäuren mutagenisiert sind, oder dass Aminosäuren in mehr als 1 β-Faltblatt mutagenisiert sind.

3. Protein nach Anspruch 1 oder 2,
**dadurch gekennzeichnet,**
**dass** oberflächenexponierte Aminosäuren in drei oberflächenexponierten Beta-Strängen in zwei antiparallelen oberflächenexponierten Beta-Faltblättern mutagenisiert sind.

4. Protein nach einem oder mehreren der vorhergehenden Ansprüche,
**dadurch gekennzeichnet,**
**dass** es ein Kristallin aus Wirbeltieren, bevorzugt Nagern, Vögeln oder Fischen, ist.

5. Protein nach einem oder mehreren der vorhergehenden Ansprüche,
**dadurch gekennzeichnet,**
**dass** es ein Alpha-, Beta- oder Gamma-Kristallin, bevorzugt ein Gamma-Kristallin, ist.

6. Protein nach einem oder mehreren der vorhergehenden Ansprüche,
**dadurch gekennzeichnet,**
**dass** es ein Gamma-II-Kristallin-Protein ist.

7. Protein nach einem oder mehreren der vorhergehenden Ansprüche,
**dadurch gekennzeichnet,**
**dass** das Protein in einem Lösungsmittel und/oder einem Bindungspartner zugänglichen Bereich des Beta-Faltblatts mutagenisiert ist.

8. Protein nach einem oder mehreren der vorhergehenden Ansprüche,
**dadurch gekennzeichnet,**
**dass** es in einer β-Faltblattstruktur einer Domäne oder einer Untereinheit des Proteins mutagenisiert ist.

9. Protein nach einem oder mehreren der vorhergehenden Ansprüche,
**dadurch gekennzeichnet,**
**dass** es mit anderen Proteinen oder Nicht-Protein-Substanzen kombiniert ist.

10. DNA, die für ein Protein nach einem oder mehreren der vorhergehenden Ansprüche kodiert.

11. RNA, die von der DNA nach Anspruch 10 abgeleitet ist.

12. Prokaryotische oder eukaryotische Vektoren oder Zellen, enthaltend eine DNA oder RNA nach Anspruch 10 oder 11 oder Teile hiervon, die für funktionelle Bereiche des Proteins kodieren.

13. Verwendung eines Proteins nach einem oder mehreren der vorhergehenden Ansprüche in der Diagnostik und Therapie, in der Kosmetik, der Bioseparation und Biosensorik und der Schadstoffverringerung.
